(19) Europäisches Patentamt
European Patent Office
Office européen des brevets

(11) Veröffentlichungsnummer: **0 230 581**
**B1**

(12) # EUROPÄISCHE PATENTSCHRIFT

(45) Veröffentlichungstag der Patentschrift: **18.04.90**

(21) Anmeldenummer: **86117010.8**

(22) Anmeldetag: **08.12.86**

(51) Int. Cl.[5]: **C 07 D 211/56**, C 07 H 5/06, A 61 K 31/445 // C07C233/36, C07C271/20, C07D491/04

(54) **3-Amino-4,5-dihydroxypiperidine, Verfahren zu ihrer Herstellung und ihre Verwendung.**

(30) Priorität: **20.12.85 DE 3545463**
**20.06.86 DE 3620645**

(43) Veröffentlichungstag der Anmeldung:
**05.08.87 Patentblatt 87/32**

(45) Bekanntmachung des Hinweises auf die Patenterteilung:
**18.04.90 Patentblatt 90/16**

(84) Benannte Vertragsstaaten:
**AT BE CH DE ES FR GB IT LI NL SE**

(56) Entgegenhaltungen:
**EP-A-0 000 947**

**CHEMISTRY LETTERS, Nr. 7, 1986, Seiten 1051-1054; G.W.J. FLEET et al.: "Synthesis of 2-acetamido-1,5-imino-1,2,5-trideoxy-D-mannitol and of 2-acetamido-1,5-imino-1,2,5-trideoxy-D-glucitol, a potent and specific inhibitor of a number of beta-N-acetylglucosaminidases"**

(73) Patentinhaber: **BAYER AG**
**D-5090 Leverkusen 1 Bayerwerk (DE)**

(72) Erfinder: **Kinast, Günther, Dr.**
**Am Eckbusch 15a**
**D-5600 Wuppertal 1 (DE)**
Erfinder: **Schüller, Matthias, Dr.**
**Gellertweg 20**
**D-5600 Wuppertal 1 (DE)**
Erfinder: **Schröder, Theo, Dr.**
**Gellertweg 38**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

3-Amino-4,5-dihydroxypiperidine, Verfahren zu ihrer Herstellung und ihre Verwendung

Die Erfindung betrifft 3-Amino-4,5-dihydroxypiperidine, Verfahren zu ihrer Herstellung und ihre Verwendung in Arzneimitteln, insbesondere zur Beeinflussung des Lipid- und Kohlehydratstoffwechsels.

Es ist bekannt, daß 1-Desoxynojirimycin eine Hemmwirkung auf Glukosidasen hat (vgl. E. Truscheit, W. Frommer, B. Junge, L. Müller, D. Schmidt, W. Wingender, Angew. Chem. *93*, 738 (1981)).

Die vorliegende Erfindung betrifft 3-Amino-4,5-dihydroxypiperidine der allgemeinen Formel (I)

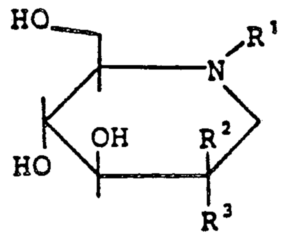

(I)

in welcher

$R^1$ für Wasserstoff, Alkyl mit bis zu 8 Kohlenstoffatomen, Aralkyl mit 7 bis 14 Kohlenstoffatomen oder für eine Gruppe der Formel

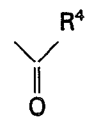

steht, wobei

$R^4$ geradkettiges oder verzweigtes Alkyl mit bis zu 8 Kohlenstoffatomen, geradkettiges oder verzweigtes Alkoxy mit bis zu 8 Kohlenstoffatomen, oder Aralkoxy mit bis zu 10 Kohlenstoffatomen bedeutet, und

$R^2$, $R^3$ für Wasserstoff oder für die Gruppe $NHR^5$ stehen, wobei

$R^5$ die gleiche Bedeutung hat wie $R^1$ und mit diesem gleich oder verschieden ist,

mit der Maßgabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

Bevorzugt sind Verbindungen der allgemeinen Formel (I) in welcher

$R^1$ für Wasserstoff, Alkyl mit bis zu 6 Kohlenstoffatomen, für Benzyl oder für eine Gruppe der Formel

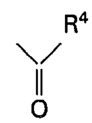

steht, worin

$R^4$ geradkettiges oder verzweigtes Alkyl mit bis zu 4 Kohlenstoffatomen, geradkettiges oder verzweigtes Alkoxy mit bis zu 4 Kohlenstoffatomen oder Benzyloxy bedeutet, und

$R^2$, $R^3$ für Wasserstoff oder für $NHR^5$ stehen, wobei

$R^5$ die gleiche Bedeutung hat wie $R^1$ und mit diesem gleich oder verschieden ist,

mit der Maßgabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

Besonders bevorzugt sind Verbindungen der allgemeinen Formel (I) in welcher

$R^1$ für Wasserstoff, Alkyl mit bis zu 4 Kohlenstoffatomen, Benzyl, Acetyl oder Benzyloxycarbonyl steht, und

$R^2$, $R^3$ für Wasserstoff oder für die Gruppe $NHR^5$ stehen, wobei

$R^5$ die gleiche Bedeutung hat wie $R^1$ und mit diesem gleich oder verschieden ist mit der Maßabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

Physiologisch unbedenkliche Salze sind im allgemeinen Salze der erfindungsgemäßen Verbindungen mit anorganischen oder organischen Säuren. Hierzu gehöhern bevorzugt Hydrochloride, Hydrobromide, Sulfate, Hydrogensulfate, Hydrogenphosphate, Hydrogencarbonate, Carbonate, Phosphate, Acetate, Maleate, Citrate, Fumarate, Oxalate oder Benzoate.

Im Rahmen der vorliegenden Erfindung umfaßt die allgemeine Formel (I) sowohl die mannokonfigurierten Derivate (Ia) als auch die glukokonfigurierten Derivate (Ib). Die D-mannokonfigurierten Derivate (Ia) stehen erfindungsgemäß für Verbindungen folgender Formel:

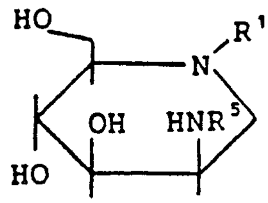

(Ia)

Die D-glukokonfigurierten Derivate (Ib) stehen erfindungsgemäß für Verbindungen folgenden Formel:

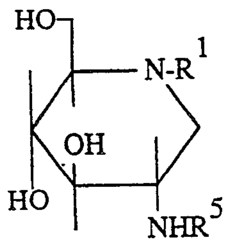

(Ib)

Die mannokonfigurierten Derivate der allgemeinen Formel (Ia)

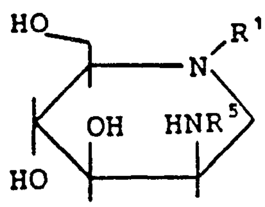

(Ia)

in welcher

$R^1$ für Wasserstoff, Alkyl mit bis zu 8 Kohlenstoffatomen, Aralkyl mit 7 bis 14 Kohlenstoffatomen, oder für die Gruppe

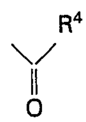

steht, worin

$R^4$ geradkettiges oder verzweigtes Alkyl mit bis zu 8 Kohlenstoffatomen, geradkettiges oder verzweigtes Alkoxy mit bis zu 10 Kohlenstoffatomen oder Aralkoxy mit bis zu 10 Kohlenstoffatomen bedeutet, und

$R^5$ die gleiche Bedeutung hat wie $R^1$ und mit diesem gleich oder verschieden ist, sowie ihre physiologisch unbedenklichen Salze erhält man, indem man die Verbindung der Formel (II)

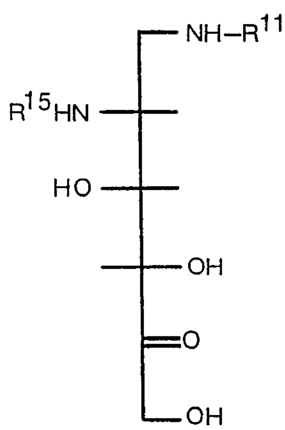

(II)

in welcher

$R^{11}$ für eine Aminoschutzgruppe, bevorzugt Benzyloxycarbonyl, Benzyl oder tert.-Butyloxycarbonyl steht und nicht Wasserstoff bedeutet, und

$R^{15}$ für eine Aminoschutzgruppe ungleich $R^{11}$, bevorzugt Acetyl steht,

in inerten Lösungsmitteln unter reduktiven Bedingungen cyclisiert, gegebenenfalls Schutzgruppen abspaltet, gegebenenfalls neue Schutzgruppen einführt und gegebenenfalls anschließend mit Säure die entsprechenden Salze herstellt.

Das Verfahren kann beispielsweise durch folgendes Schema verdeutlicht werden:

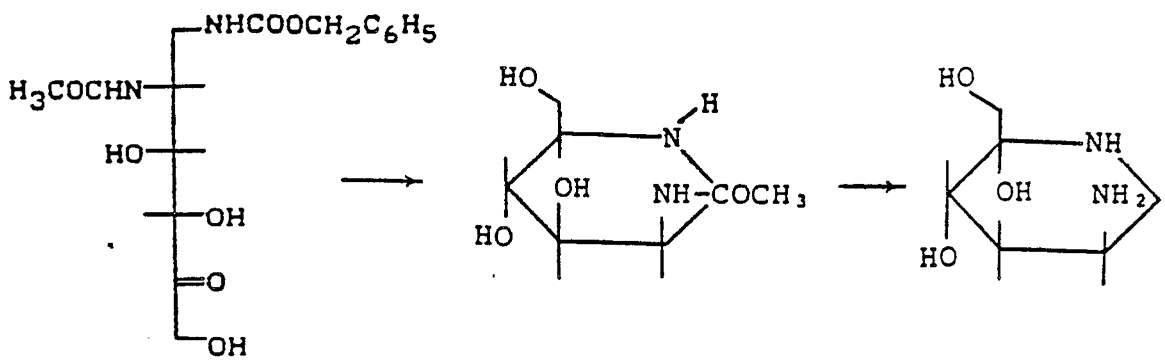

Die reduktive Cyclisierung wird im allgemeinen in Anwesenheit eines Reduktionsmittels, gegebenenfalls in Anwesenheit eines Katalysators durchgeführt.

Bevorzugt führt man die Cyclisierung mit Wasserstoff als Reduktionsmittel durch. Als Katalysatoren eignen sich hierbei Edelmetallkatalysatoren wie Pd, Pt, bevorzugt Pd auf Tierkohle.

Als Lösungsmittel eignen sich hierbei Wasser und alle inerten organischen Lösungsmittel, die sich unter den Reaktionsbedingungen nicht verändern. Bevorzugt gehören hierzu Alkohole wie Methanol, Ethanol, Propanol oder Isopropanol, Ether wie Diethylether, Dioxan, Tetrahydrofuran oder Glykoldimethylether, Dimethylformamid oder Eisessig. Ebenso ist es möglich, Gemische der genannten Lösungsmittel einzusetzen.

Im allgemeinen wird die Reaktion bei einem Wasserstoffdruck von 1 bis 150 bar, bevorzugt von 1 bis 100 bar durchgeführt.

Im allgemeinen wird die Reaktion in einem Temperaturbereich von 20 bis 150°C, bevorzugt von 20 bis 100°C durchgeführt.

Als Reduktionsmittel eignen sich weiterhin Alkalimetallcyanborhydride, Dialkylaminoborane oder Alkalimetallborhydride.

Als Lösungsmittel können die selben Lösungsmittel verwendet werden, wie bei der Cyclisierung mit Wasserstoff. Besonders geeignet sind hier Mischungen von Wasser mit Alkoholen oder Dimethylformamid.

Die reduktive Cyclisierung wird im allgemeinen bei Normaldruck und in einem Temperaturbereich von 0°C bis 150°C, bevorzugt von 20°C bis 100°C durchgeführt.

Das erfindungsgemäße Verfahren wird im allgemeinen in einem pH-Bereich von 1 bis 11, bevorzugt von 4 bis 9 durchgeführt, je nach Art der vorhandenen Schutzgruppen $R^1$, die bei der reduktiven Cyclisierung in situ abgespalten wird, ohne daß gleichzeitig $R^5$ abgespalten wird.

Die Abspaltung von Schutzgruppen bzw. die Einführung neuer Schutzgruppen erfolgt im allgemeinen nach üblichen Methoden, wie sie beispielsweise in Houben-Weyl's "Methoden der organischen Chemie" XV/1 und 2 beschrieben sind und dem Fachmann vertraut sind.

Die als Ausgangsstoffe eingesetzten Verbindungen der allgemeinen Formel (II) sind neu.

Sie können nach an sich bekannten Methoden z.B. gemäß folgendem Schema hergestellt werden:

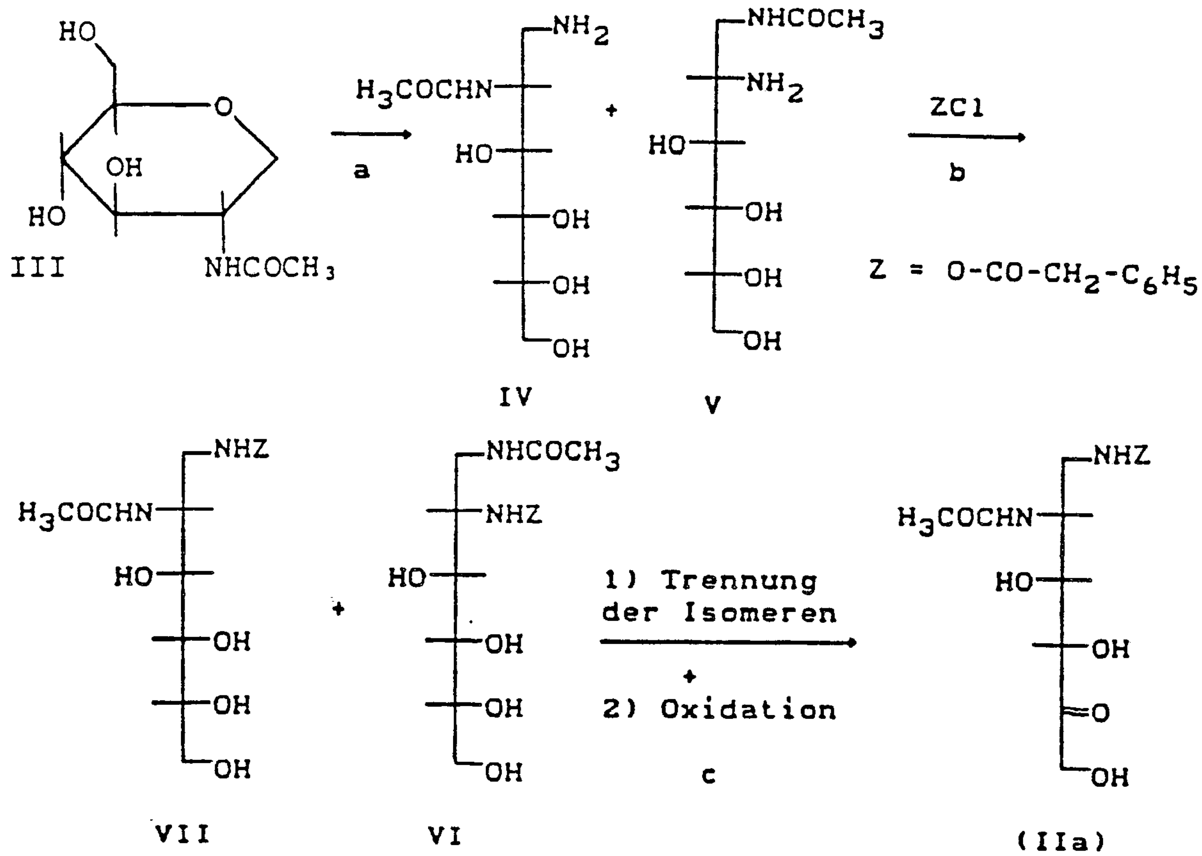


Hierbei wird in Schritt a N-Acetylglucosamin III in inerten Lösungsmitteln wie z.B. Wasser, Alkoholen, z.B. Methanol, Ethanol, Propanol, Isopropanol oder Gemische der genannten Lösungsmittel in Anwesenheit eines Katalysators wie Raney-Nickel, in Gegenwart von Ammoniak bei Temperaturen von 20 bis 200°C, bevorzugt von 1 bis 150°C, bei einem Druck von 1 bis 200, bevorzugt von 1 bis 150 bar hydriert, wobei die Mischung de offenkettigen isomeren Verbindungen IV und V entsteht. Diese Mischung wird ohne vorherige Trennung in Schritt b in inerten Lösungsmitteln wie Wasser oder Alkoholen, z.B. Methanol, Ethanol, Propanol oder Isopropanol oder deren Gemischen, Anwesenheit von einer Base wie Natrium- oder Kaliumcarbonat, bei Temperaturen von −20°C bis +80°C, bevorzugt von 0°C bis 40°C mit Benzyloxycarbonylchlorid umgesetzt und das Produktgemisch aus VI und VII nach üblichen Verfahren der Chromatographie getrennt. Im letzten Schritt c wird schließlich das mannokonfigurierte Isomere nach an sich bekannten Methoden zur Verbindung II oxidiert, bevorzugt nach einem mikrobiologischen Verfahren unter Verwendung von Glucobacter-Zellen, wie es beispielsweise von G. Kinast, M. Schedel in Angew. Chem. Int. Ed. *20* (9) 805 (1981) beschrieben ist.

Die glukokonfigurierten Derivate der allgemeinen Formel (Ib)

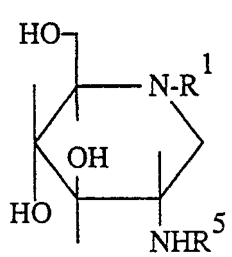

(Ib)

in welcher

$R^1$ für Wasserstoff, Alkyl mit bis zu 8 Kohlenstoffatomen, Aralkyl mit 7 bis 14 Kohlenstoffatomen oder für die Gruppe

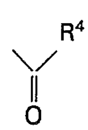

steht, worin

$R^4$ geradkettiges oder verzweigtes Alkyl mit bis zu 8 Kohlenstoffatomen, geradkettiges oder verzweigtes Alkoxy mit bis zu 8 Kohlenstoffatomen, oder Aralkoxy mit bis zu 10 Kohlenstoffatomen bedeutet, und

$R^5$ die gleiche Bedeutung hat wie $R^1$ und mit diesem gleich oder verschieden ist, sowie ihre physiologisch unbedenklichen Salze, erhält man, indem man Azidoverbindungen der Formel (VIII)

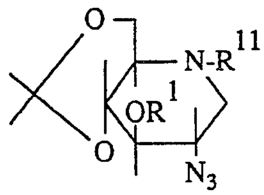

(VIII)

in welchen

$R^{11}$ für eine Aminoschutzgruppe, bevorzugt für Benzyl steht,

in inerten Lösungsmitteln in Aswesenheit eines Reduktionsmittel, gegebenenfalls in Anwesenheit eines Katalysators reduziert, gegebenenfalls Schutzgruppen abspaltet, gegebenenfalls neue Schutzgruppen $R^5$ einführt und gegebenenfalls anschließend mit Säure die entsprechenden Salze herstellt.

Die Reduktion wird im allgemeinen nach den Methoden durchgeführt, wie sie für die Reduktion von Aziden zu Aminogruppen üblich ist.

Hierzu gehören die Reduktion mit Wasserstoff als Reduktionsmittel, in Anwesenheit eines Katalysators wie Raney-Nickel, Pd, Pt (ggfs. auf Tierkohle), gegebenenfalls in Anwesenheit von Säuren wie Sulfonsäuren (z.B. Methan-, Benzol-, Toluolsulfonsäure), oder Mineralsäuren (z.B. HCl, $H_2SO_4$, HBr) oder Carbonsäuren (Essigsäure, Trifluoressigsäure (in inerten Lösungsmitteln wie Wasser, Alkoholen, z.B. Methanol, Ethanol, Propanol, Isopropanol, Dimethylformamid oder Gemische derselben, oder die Reduktion mit Hydriden als Reduktionsmittel wie beispielsweise Natriumborhydrid, Lithiumalanat oder Natriumcyanoborhydrid, gegebenenfalls mit Nickelsalzen in inerten Lösungsmitteln wie Wasser, Alkoholen wie Methanol, Ethanol, Propanol, Isopropanol oder Dimethylformamid, oder Gemischen der genannten Lösungsmittel, sowei die Reduktion mit Schwefelwasserstoff in Pyridin.

Weitere erfindungsgemäß anwendbare Reduktionsverfahren für Azide werden in Houben-Weyl's "Methoden der organischen Chemie" X/3, 822, XI/1, 539 beschrieben.

Die Abspaltung von Schutzgruppen bzw. Neueinführung neuer Schutzgruppen erfolgt im allgemeinen nach üblichen Methoden wie sie z.B. in Houben-Weyl's "Methoden der organischen Chemie" XV/1 und 2 sowie Baker und Ollis' "Comprehensive organic Chemistry" *5*, 714 beschrieben sind. Sie sind bekannt und dem Fachmann vertraut.

Die als Ausgangsstoffe verwendeten Azidoverbindungen der Formel VIII sind neu. Sie können jedoch nach im Prinzip bekannten Methoden gemäß folgendem Schema hergestellt werden:

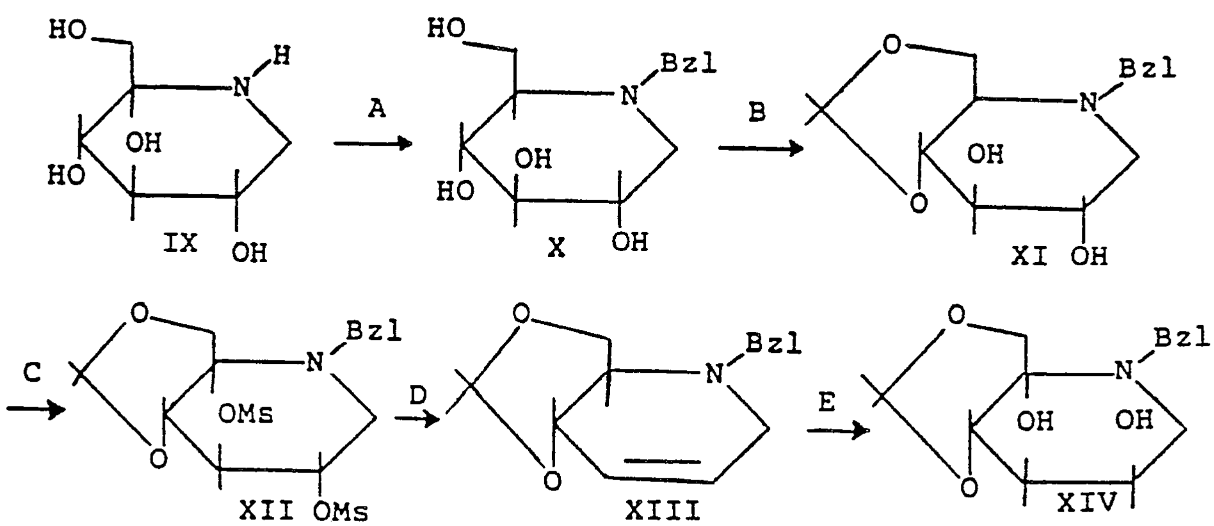


IX X XI XII XIII XIV

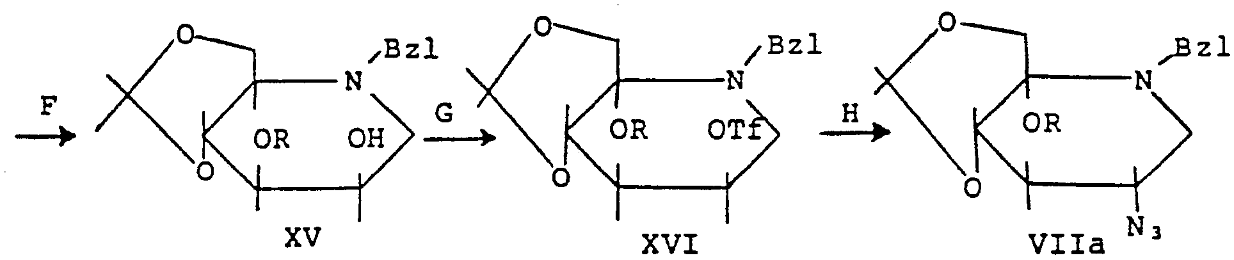

Bzl. = $CH_2C_6H_5$
Ms = Mesyl
Tf = Trifluormethylsulfonyl
R = Bzl. oder p-Methoxy-benzyl

Danach wird in Schritt A Desoxynojirimycin (bekannt aus EP—OS 947) mit Benzylbromid und Kaliumcarbonat in Dimethylformamid bei Eiskühlung zu X benzyliert. In Schritt B wird die Benzylverbindung X nach Abtrennung des Kaliumcarbonats mit Säuren (pH 2) und 2-Methoxypropen oder 2,2-Dimethoxypropan in Dimethylformamid zu XI umgesetzt und XI in Schritt C mit Methansulfonsäurechlorid in Aceton/Triethylamin in das Mesylat XII überführt. Durch Umsetzung von XII mit NaI/Zn in Dimethylformamid (Schritt D) erhält man das Olefin XIII, das anschließend (Schritt E) mit $OsO_4$/N-Methylmorpholin-N-oxid zu XIV hydroxyliert wird. Das mannokonfigurierte Derivat XIV wird in Schritt F über ein in situ erzeugtes Bis-dibutylzinnacetal stereoselektiv it Benzylbromid oder auch p-Methoxybenzylchlorid und Tetrabutylammoniumbromid zu XV benzyliert.

XV wird in Schritt G mit Trifluormethansulfonsäureanhydrid in $CH_2Cl_2$/Pyridin bei −20°C in XVI überführt und XVI anschließend (Schritt H) mit $LiN_3$ in einem $CH_2Cl_2$/Dimethylformamid — Gemisch bei −40°C unter Inversion an C—2 stereoselektiv in das Azid VIIa überführt.

Die erfindungsgemäßen Verbindungen haben interessante pharmakologische Wirkungen. Sie hemmen den extra- und intrazellulären Kohlehydratstoffwechsel, Glykosyltransferasen sowie Glukosidasen. Darüberhinaus beeinflussen sie die Glykoprotei- sowie Chitinbiosynthese und wirken fungizid, bakteriostatisch und als Immunmodulatoren.

Sie können aufgrund ihrer Eigenschaften beispielsweise als Therapeutika für folgende Erkrankungen eingesetzt werden: Prädiabetes, Gastritis, Obstipation, Karies, Atherosklerose, Adipositas, Diabetes oder Hyperlipoproteinämie.

Die Verbindungen können ohne Verdünnung, z.B. als Pulver oder in einer Gelatinehülle oder in Kombination mit einem Trägerstoff in einer pharmazeutischen Zusammensetzung appliziert werden. Pharmazeutische Zusammensetzungen können eine größere oder kleinere Menge des Wirkstoffes enthalten, z.B. 0,1% bis 99,5%, in Kombination mit einem pharmazeutisch verträglichen nicht-toxischen, inerten Trägerstoff, wobei der Trägerstoff eine oder mehrere feste, halbfeste oder flüssige Verdünnungsmittel, Füllstoffe und/oder nichttoxische, inerte und pharmazeutisch verträgliche Formulierungshilfsmittel enthalten kann. Solche pharmazeutischen Zubereitungen liegen bevorzugt in Form von Dosierungseinheiten vor, d.h. physikalisch-diskreten, eine bestimmte Menge des Wirkstoffes enthaltenden Einheiten, die einem Bruchteil oder einem Vielfachen der Dosis entsprechen, die zur Herbeiführung der gewünschten Wirkung erforderlich sind. Die Dosiseinheiten können 1,2,3,4 oder mehr Einzeldosen oder 1/2, 1/3 oder 1/4 einer Einzeldosis enthalten. Eine Einzeldosis enthält vorzugsweise eine genügende Menge Wirkstoff, um bei unter Applikation gemäß eines vorher bestimmten Dosierungsschemas einer oder mehrerer Dosierungseinheiten die gewünschte Wirkung zu erzielen, wobei eine ganze, eine halbe, ein Drittel oder ein Viertel der Tagesdosis gewöhnlich zu allen, Haupt- und Nebenmahlzeiten am Tag verabreicht wird.

Andere therapeutische Mittel können auch eingenommen wrden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, z.B. als Pulver, Tabletten, Dragees, Kapseln, Granulate, Suspensionen, Lösungen und dergleichen.

Pulver wird durch Zerkleinerung der Substanz zu einer geeigneten Größe und Vermischen mit einem ebenfalls zerkleinerten pharmazeutischen Trägerstoff hergestellt. Obgleich ein eßbares Kohlenhydrat, wie z.B. Stärke, Lactose, Saccharose oder Glukose normalerweise zu diesem Zweck Verwendung findet und auch hier benutzt werden kann, ist es wünschenswert, ein Kohlehydrat, das nicht metabolisiert wird, wie z.B. ein Cellulosederivat, zu benutzen.

Süßmittel, Geschmackzusätze, Konservierungsstoffe, Dispergiermittel und Färbemittel können auch mitverwendet werden.

Die Kapseln können durch Zubereitung der oben beschriebenen Pulvermischungen und durch Füllung bereits gebildeter Gelatinehüllen hergestellt werden. Die Pulvermischungen kann man vor dem

Füllvorgang mit Gleitmitteln, wie z.B. Kieselgel, Talkum, Magnesiumstearat, Calciumstearat oder festem Polyethylenglykol versetzen.

Die Mischung kann ebenfalls mit einem Desintegrator oder Lösungsvermittler, wie z.B. Agar-Agar, Calciumcarbonat oder Natriumcarbonat versetzen, um bei Einnahme der Kapsel die Zugänglichkeit des Wirkstoffes zu vebessern.

Die Antfertigung der Tabletten erfolgt z.B. durch Herstellen einer Pulvermischung, grob oder feinkörnig, und Hinzufügen eines Gleitmittels und Desintegrators. Aus dieser Mischung formt man Tabletten. Eine Pulvermischung bereitet man vor durch Mischung der Substanz, welche in geeigneter Weise zerkleinert wurde, und ergänzt ein Verdünnungsmittel oder eine andere Trägersubstanz wie oben beschrieben. Gegebenenfalls füht man ein Bindemittel, z.B. Carboxymethylcellulose, Alginate, Gelatine oder Polyvinylpyrrolidone, einen Lösungsverzögerer, wie z.B. Paraffin, einen Resorptionsbeschleuniger, wie z.B. ein quarternäres Salz und/oder ein Adsorptionsmittel, wie z.B. Bentonit, Kaolin oder Dicalciumphosphat hinzu. Die Pulvermischung kann granuliert werden zusammen mit einem Bindemittel, wie z.B. Sirup, Stärkepaste, Akazienschleim oder Lösungen aus Zellulose- oder Polymerenmaterial. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den Düsen stecken bleiben, kann man sie mit einem Gleitmittel versetzen, wie z.B. Stearinsäure, Stearatsalz, Talkum oder Mineralöl. Diese gleitfähig gemachte Mischung wird dann in Tablettenform gepreßt. Die Wirkstoffe können auch mit freifließenden, inerten Trägerstoffen vereinigt werden und direkt in Tablettenform gebracht werden unter Auslassung der Granulat- und Zerstückelungsschritte. Mann kann das Produkt mit einer klaren oder opaken Schutzhülle versehen, z.B. einem Überzug aus Schellack, einem Überzug aus Zucker oder Polymersubstanzen und einer polierten Hülle aus Wachs. Farbstoffe können diesen Überzügen beigefügt werden, damit zwischen den einzelnen Dosierungseinheiten unterschieden werden kann.

Die oral verabreichten Zubereitungsformen, z.B. Lösungen, Sirupe und Elixiere, lassen sich in Dosierungseinheiten herstellen, so daß eine bestimmte Menge Präparat eine bestimmte Menge Wirkstoff enthält. Sirup kann so hergestellt werden, daß der Wirkstoff in einer wäßrigen Lösung, welche geeignete Geschmacksstoffe enthält, gelöst wird. Elixiere werden unter Verwendung nichttoxischer, alkoholischer Trägerstoffe erhalten. Suspensionen kann man durch Dispergieren der Verbindung in einem nichttoxischen Trägerstoff darstellen. Lösungsvermittler und Emulgiermittel, wie z.B. ethoxylierte Isostearylalkohole und Polyoxyethylensorbitester, Konservierungsmittel, geschmacksverbessernde Zusätze, wie z.B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z.B. durch Einhalten des Wirkstoffes in Polymersubstanzen, Wachse und degleichen.

Zusätzlich zu den oben angegebenen pharmazeutischen Zusammensetzungen lassen sich auch diese enthaltende Lebensmittel herstellen, beispielsweise Zucker, Brot, Kartoffelprodukte, Fruchtsaft, Bier, Schokolade oder andere Konfektartikel und Konserven, wie beispielsweise Marmelade, wobei zu diesen Produkten eine wirksame Menge mindestens eines der erfindungsgemäßen Wirkstoffe gegeben wird.

Die unter Verwendung der erfindungsgemäßen Wirkstoffe hergestellten Nahrungsmittel eignen sich sowohl zur Diät bei Patienten, die an Stoffwechselstörungen leiden, als auch zur Ernährung gesunder Personen im Sinne einer Stoffwechselstörung vorbeugenden Ernährugsweise.

Die erfindungsgemäßen Verbindungen weisen weiterhin die Eigenschaften auf, in Tieren das Verhältnis des Anteils an ungewünschtem Fett zum Anteil des gewünschten fettarmen Fleisches zugunsten des mageren Fleisches in hohem Maß zu beeinflussen. Dies ist von besonderer Bedeutung für die Aufzucht und Erhaltung von sonstigen Nutz- und Ziertieren. Die Verwendung der Wirkstoffe kann weiterhin erheblich zur Rationalisierung der Fütterung der Tiere führen, sowohl zeitmäßig, mengenmäßig als auch qualitätsmäßig. Da sie eine gewisse Verzögerung der Verdauung bewirken wird die Verweildauer der Nährstoffe im Verdauungstrakt verlängert, wodurch eine mit weniger Aufwand verbundene ad libitum Fütterung ermöglicht wird. Weiterhin ergibt sich bei der Verwendung der erfindungsgemäßen Stoffe in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowei zur Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlägerung neigen.

Herstellungsbeispiele

Beispiel 1

2-Acetamido-1-amino-1,2-didesoxy-D-mannit

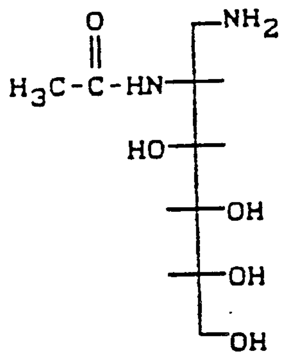

221 g (1 mol) Acetylglucosamin wurden in 2 l Methanol gelöst, 20 g Raney-Nickel und 250 ml $NH_3$ konz. hinzugegeben und 5 Stunden bei 100°C mit 100 bar $H_2$ hydriert. Zur Aufarbeitung wird vom Katalysator abgesaugt und der Ansatz zur Trockene eingedampft. Das erhaltene Rohprodukt wird ohne weitere Reinigung wie in Beispiel 2 angegeben umgesetzt.

Beispiel 2

2-Acetamido-1-benzyloxycarbonylamino-1,2-didesoxy-D-mannit

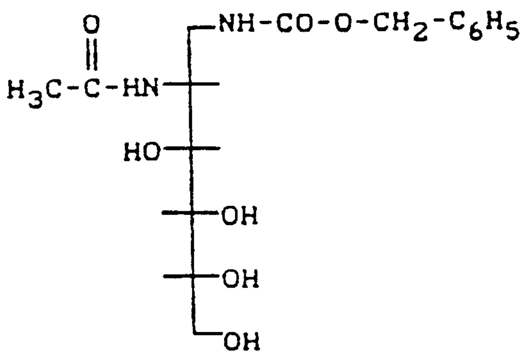

Das Rohprodukt aus Beispeil 1, das durch intensives Trokknen im Vakuum und über $H_2SO_4$ konz. frei von $NH_3$ ist, wird in 250 ml Wasser gelöst, unter Eiskühlung und Rühren 150 g (1,1 mol) Benzyloxycarbonylchlorid und 150 g (1,1 mol) $K_2CO_3$ zugegeben und der Anzatz 18 Stunden bei Raumtemperatur gerührt. Anschließend wird vom Niederschlag abgesaugt, das Filtrat zur Trockene eingedampft und der Rückstand an RP8 (Laborsäule der Fa. Merck, Größe C) aufgetrennt. Hierzu werden 2 Säulen hintereinander geschaltet, 20 g des Rückstandes in etwa 200 ml $H_2O$ gelöst, die erhaltene Lösung auf die Säule aufgetragen und anschließend bei einem Fluß von etwa 15 ml/min mit 1,5 l $H_2/CH_3CN$ 90/10 und 1,5 l $H_2O/CH_3CN$ 80/20 eluiert. Die Fraktionen werden per HPLC untersucht (Säule: Li Chrosorb/RP8 (10 µm), 2 ml/min Puffer pH 7/ $CH_3CN$ 80/20, 210 nm) und die Fraktionen mit einer Retentionszeit von 3,35 min zusammengegeben, das Acetonitril im Vakuum abgezogen und der Rückstand lyophylisiert. Aus 20 g des aufgetragenen Rückstandes erhält man 1 bis 2 g des reinen Produktes.

Beispiel 3

5-Acetamido-6-benzyloxycarbonylamino-5,6-didesoxy-D-fructose

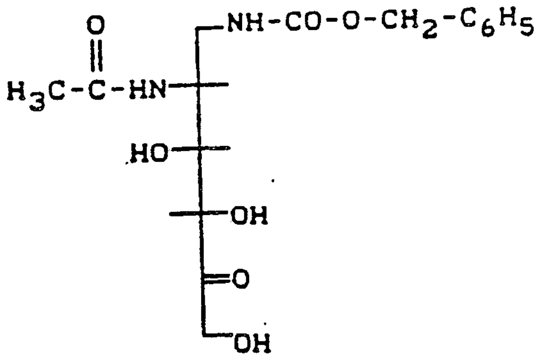

4 g Glucobacter Zellen werden in 100 ml 0,1 M Phosphatpuffer pH 4,5 (mit Leitungswasser angesetzt) suspendiert und mit 1,2 g Acetamido-1-benzyloxycarbonylamino-1,2-didesoxy-D-mannit versetzt. Man läßt über Nacht bei 32°C schütteln. Nach 30 Stunden wird abzentrifugiert und der Rückstand zweimal mit 100 ml Methanol gewaschen. Der Überstand und die Methanol-Extrakte werden vereinigt und zur Trockene eingedampft. Das Rohprodukt wird ohne Aufarbeitung weiterverarbeitet.

Beispiel 4

2-Acetamido-1,5-imino-1,2,5-tridesoxy-D-mannit

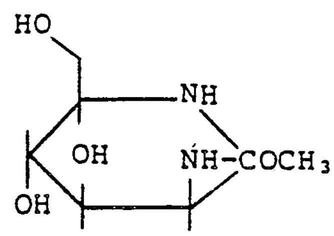

0,6 g des Produktes aus Beispiel 3, 1,1 g Pd/C (5%ig) und 3 mg $K_2CO_3$ werden in 25 ml $H_2O$ und 50 ml Methanol gelöst und 4 Stunden bei 60°C mit 50 atm hydriert. Zur Aufarbeitung wird der Katalysator abgesaugt, das Filtrat über eine Säule mit einem sauren Ionenaustauscher (Lewatit SP 112, H-Form) gegeben, mit $H_3COH/H_2O$ 80/20 gewaschen und anschließend das Rohprodukt mit $H_3COH/NH_3$ konz. 80/20 eluiert, die das Produkt enthaltenen Fraktionen (DC-System: $CHCl_3/CH_3OH/NH_3$ konz. 4:3:1) einrotiert und aus $H_2O/H_3COH$ umkristallisiert. Ausbeute 0,21 g.

Fig. 1 zeigt das $^1$H-NMR-Spektrum der Verbindung des Beispiels 4 (250 MHz/$D_2O$).

Beispiel 5

2-Aimino-1,5-imino-1,2,5-tridesoxy-D-mannit

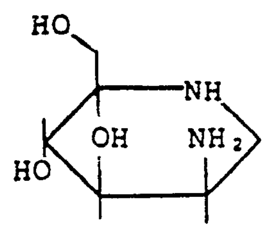

Die Verbindung aus Beispiel 4 (0,5 g) wurde in 2N NaOH 2 Stunden zum Rückfluß erhitzt, neutralisiert mit 2N HCl und wie in Beispiel 3 angegeben über einen sauren Ionenaustauscher greinigt.

Ausbeute: 0,2 g.

Beispiel 6

N-Benzyl-1,5-didesoxy-1,5-imino-D-glucit

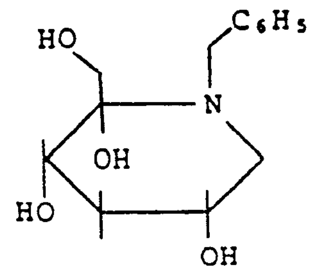

163,2 g (1,0 mol) Desoxynojirimycin werden in 2,5 l Dimethylformamid gelöst. Der Lösung werden 76,0 g (0,55 mol) Kaliumcarbonat zugesetzt. Nach Abkühlung auf 0°C tropft man während 30 Minuten 188,1 g (1,1 mol) Benzylbromid zu. Nach etwa 120 Min. ist das Ausgangsprodukt vollständig umgesetzt. Das Produkt wird in situ weiterverarbeitet.

Ein kleiner Teil wird zur Charakterisierung abgetrennt, nach Entfernung des Lösungsmittels säulenchromatographisch an Kieselgel im System Chloroform/Methanol = 4:1 gereinigt und als kristalliner Feststoff erhalten.

Fp.: 181°C $[\alpha]_D^{20}$ = −48,30° (Methanol)

Beispiel 7

N-Benzyl-4,6-O-isopropyliden-1,5-didesoxy-1,5-imino-D-glucit

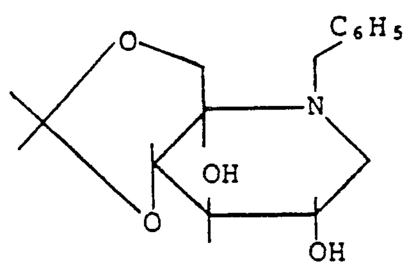

Dem Reaktionsgemisch aus Beispiel 6 wird nach Abfiltrieren des Bodenkörpers soviel p-Toluolsulfonsäure zugesetzt, bis sich ein pH von 1,5 einstellt. Sodann wird die Mischung aus 208 g (2 mol) Dimethoxypropan und 144 g (2 mol) 2-Methoxy-propen zugesetzt. Nach 2 Stunden Reaktionszeit bei 30°C wird das Reaktionsende durch Dünnschichtchromatographie in Toluol:Ethanol = 3:1 festgestellt. Die Aufarbeitung erfolgt durch mehrstündiges Rühren mit einer Suspension von 210 g Natriumhydrogencarbonat in 100 ml Wasser. Nach Verdampfen des Lösungsmittels wird der Rückstand mit Chloroform digeriert, die Chloroformphase gewaschen, über Natriumsulfat getrocknet und nach Verdampfen des Lösungsmittels der Rückstand aus Essigester/Petrolether umkristallisiert.

Ausbeute: 249,3 g.

Fp.: 117°C $[\alpha]_D^{20}$ = 100,3° (Methanol).

Beispiel 8

N-Benzyl-4,6-O-isopropyliden-2,3-di-O-mesyl-1,5-didesoxy-1,5-imino-D-glucit

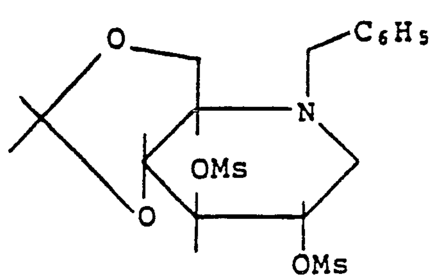

Ms = Mesyl
= $SO_2CH_3$

240 g (0,81 mol) des Produktes aus Beispiel 7 werden in 2000 ml Aceton und 300 ml Triethylamin (2,1 mol) gelöst. Hierzu wird langsam unter Eiskühlung eine Lösung von 138 ml (1,8 mol) Methansulfonsäurechlorid in 600 ml Aceton getropft. Anschließend wird die Kühlung entfernt und der

Ansatz über Nacht bei Raumtemperatur gerührt. Nach vollständiger Umsetzung (DC Toluol/Aceton 4:1) wird zunächst von den ausgefallenen Salzen abfiltriert, sodann überschüssiges Mesylchlorid mit wenig Eis hydrolysiert und der Ansatz am Rotationsverdampfer eingeengt. Der kristalline Rückstand wird in Methylenchlorid aufgenommen, mit Wasser gewaschen, die organische Phase wird über $Na_2SO_4$ getrocknet uind eingeengt.

Das Produkt ist für die weitere Umsetzung sauber genug (≥ 97%), kann aber aus Essigester/Petrolether umkristallisiert werden.

Ausbeute: 354 g (97% der Theorie).

Fp.: 162°C $[\alpha]_D^{20}$ = −44,8° ($CHCl_3$).

Beispiel 9

N-Benzyl-4,6-O-isopropyliden-1,2,3,5-tetradesoxy-1,5-imino-D-erythro-hex-2-enit

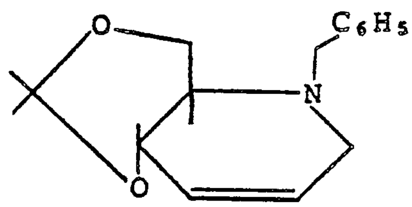

90 g (0,2 mol) des Produktes aus Beispiel 8 werden in 1600 ml Dimethylformamid gelöst, nach Zusatz von 149,9 g (1,0 mol) NaI und 130,8 g (2,0 mol) Zn-Pulver 3 Stunden bei 135°C gerührt (DC: Toluol/EE 3:1). Nach dem Abkühlen wird von den Salzen abgesaugt, der gesamte Ansatz eingeengt, in $CH_2Cl_2$ aufgenommen und mehrfach mit Wasser gewaschen. Die organische Phase wird über $Na_2SO_4$ getrocknet, eingeengt und der verbleibende Sirup über Kieselgel filtriert (230—400 mesh; Laufmittel: Toluol; Toluol/Aceton 30:1).

Ausbeute: 36,3 g (70% der Theorie).

$[\alpha]_D^{20}$ = −51,4° ($CHCl_3$).

Beispiel 10

N-Benzyl-4,6-O-isopropyliden-1,5-didesoxy-1,5-imino-D-mannit

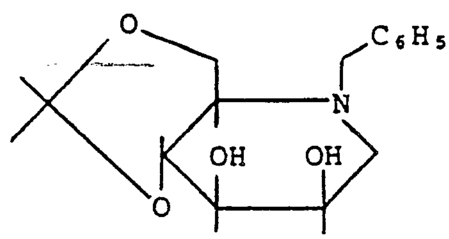

40 g (0,154 mol) des Produktes aus Beispiel 9 werden in 1200 ml Aceton, 300 ml $H_2O$ und 100 ml tert.-Butanol gelöst und nach Zugabe von 27,0 g (0,2 mol) N-Methylmorpholin-N-oxid-Hydrat und 400 mg $OsO_4$ solange gerührt, bis dünnschichtchromatographisch (Toluol/Aceton 1:1) vollständige Umsetzung beobachtet wird. Der Reaktionsansatz wird dann mit 10 ml Cyclohexen versetzt und noch 2 Stunden gerührt (Schwarzfärbung). Die Lösung wird dann über Cellite® filtriert und eingeengt. Der sirupöse Rückstand wird in $CH_2Cl_2$ aufgenommen, mehrfach mit Wasser gewaschen, die organische Phase über $Na_2SO_4$ getrocknet und erneut eingeengt. Der Rückstand wird durch Flash-Chromatographie an Kieselgel gereinigt (Laufmittel: Toluol/Essigester 1:1 bis 1.3) und aus Essigester/Petrolether kristallisert.

Ausbeute: 33,9 g (75%)

Fp.: 123—125°C $[\alpha]_D^{20}$ = 122,8 ($CHCl_3$).

Beispiel 11

N-Benzyl-3-O-benzyl-4,6-O-isopropyliden-1,5-didesoxy-1,5-imino-D-mannit

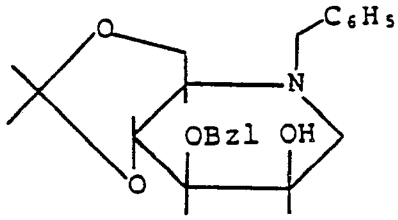

Eine Lösung von 15 mg (51 mmol) der Verbindung des Beispiels 10 in 300 ml abs. Toluol in der 14 g (56 mmol) Dibutylzinnoxid suspendiert sind, wird unter $N_2$ Schutzgasatmosphäre und Anwendung eines Wasserabscheiders solange zum Rückfluß erhitzt, bis keine Wasserabscheidung merhr zu beobachten ist. Sodann wird die Temperatur auf 100°C gesenkt und der Lösung im Stickstoffgegenstrom 1,64 g (5,1 mmol) Tetrabutylammoniumbromid zugesetzt. Nachfolgend werden 9,58 g (56 mmol) Benzylbromid während 5 Stunden langsam zugetropft. Nach 8 Stunden wird dünnschichtchromatographisch (System Toluol:Aceton = 5:1) nahezu vollständige Umsetzung des Eduktes festgestellt und aufgearbeitet. Dies erfolgt durch Verdampfen des Lösungsmittels, Aufnehmen des Rückstandes in Chloroform und nachfolgendes Waschen der organischen Phase mit gesättigter Natriumhydrogencarbonatlösung. Dabei anfallende Zinnsalze wurden durch Zentrifugieren abgetrennt. Das nach Trocknen über Natriumsulfat und Verdampfung des Lösungsmittels erhaltene Rohprodukt wird säulenchromatographisch im System Toluol:aceton = 40:1 von begleitenden Nebenprodukten befreit.

Ausbeute: 14,3 g (37 mmol; 73% der Theorie)

$[\alpha]_D^{20} = -55{,}3°$; = 0,85; (Ethanol).

Fig. 2 zeigt das $^1$H-NMR-Spektrum der Verbindung des Beispiels 11 (200 MHz, $CDCl_3$).

Fig. 3 zeigt das IR-Spektrum der Verbindung des Beispiels 11 ($CHCl_3$).

Fig. 4 zeigt das Massenspektrum der Verbindung des Beispiels 11.

Beispiel 12

N-Benzyl-3-O-benzyl-4,6-O-isopropyliden-2-trifluormethansulfonyl-1,5-didesoxy-1,5-imino-D-mannit

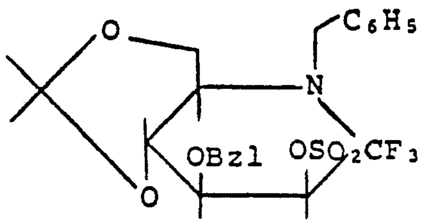

14 g (37 mmol) der Verbindung Beispiels 11 werden in 140 ml abs. Dichlormethan gelöst. Die Lösung wird mit 6,9 g (88 mmol) Pyridin versetzt und unter Inertgasatmosphäre auf −20°C thermostatisiert. Unter diesen Bedingungen werden 10,4 g (37 mmol) Trifluormethansulfonsäureanhydrid gelöst in 60 ml abs. Dichlormethan über 60 min. zugetropft. Nach 1 Stunde Reaktionszeit ist das Ausgangsprodukt in Endprodukt übergegangen. ($R_f$ = 0,3, Toluol:Aceton = 10:1).

Die Aufarbeitung erfolgt durch Zugabe von 50 ml gesättigter Natriumhydrogencarbonatlösung. Nach 30 Minuten heftigem Rühren bei 0°C wird die organische Phase über Natriumsulfat getrocknet und eingeengt. Man erhält 18,1 g (35 mmol; 95%) Produkt, das sogleich dem nächsten Reaktionsschritt unterworfen wird.

Beispiel 13

2-Azido-N-benzyl-3-O-benzyl-4,6-O-isopropyliden-1,2,5-tridesoxy-1,5-imino-D-glucit

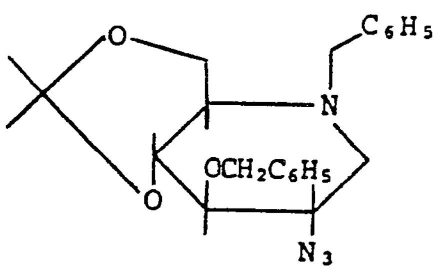

18,1 g (35 mmol) der Verbindung aus Beispiel 12 werden unter Schutzgasatmosphäre in 40 ml abs. Dichlormethan gelöst. Man thermostatiert auf −40°C und setzt langsam 100 ml abs. Dimethylformamid zu. Sodann versetzt man mit 8,6 g (0,175 mol) Lithiumazid. Das Gemenge wird bei −40°C unter Schutzgas gerührt. Nach etwa 8 h ist dünnschichtchromatograpisch kein Edukt mehr nachzuweisen. (Sprühreagens: Naphthoresorcin).

Die Aufarbeitung erfolgt durch Einengen bei 30°C Badtemperatur im Hochvakuum auf etwa 1/4 des Ausgangsvolumens, Aufnehmen des Rückstandes in Toluol und mehrfaches Waschen mit verdünnter NaCl-Lösung. Nach Trocknung über Natriumsulfat und säulenchromatographischer Reinigung des Produktes im System Toluol:Essigester = 50:1 erhält man 10,6 (26,5 mmol; 74%) des reinen Produktes.

$[\alpha]_D^{20} = -78{,}86°$.

Fig. 5 zeigt das $^1$H-NMR-Spektrum der Verbindung des Beispiels 13 (300 MHz, $CDCl_3$).

Fig. 6 zeigt das IR-Spektrum der Verbindung des Beispiels 13 ($CHCl_3$).

Beispiel 14 und 15

2-Amino-N-benzyl-3-O-benzyl-4,6-isopropyliden-1,2,5-tridesoxy-1,5-imino-D-glucit *14* und 2-Acetamido-N-benzyl-4,6-idopropyliden-1,2,5-tridesoxy-1,5-imino-D-glucit *15*.

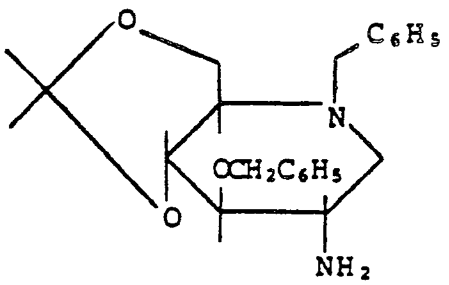

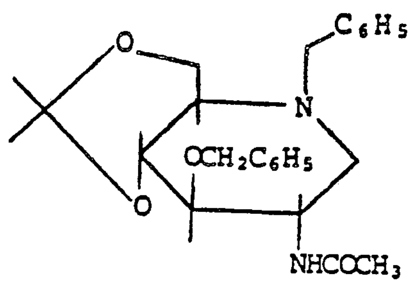

10,6 g (26 mmol) der Verbindung des Beispiels 13 werden in 100 ml einer 4%igen ethanolischen $NiCl_2$-Lösung aufgenommen und unter Rühren bei 0°C solange mit einer gesättigten ethanolischen Lösung von $NaBH_4$ versetzt, bis die anfänglich reversible Schwarzfärbung bestehen bleibt. Die dünnschichtchromatographische Kontrolle (Tol: Ethanol = 10:1) zeigt dann, daß das Edukt vollständig in die 2-Amino-Verbindung 14 überführt worden ist. (Ninhydrinientwicklung: Rotfärbung). Die Aufarbeitung erfolgt durch Rotationsverdampfung des Lösungsmittels bei 30°C Badtemperatur im Hochvakuum und nachfolgender in situ Acetylierung durch Aufnehmen in 50 ml Acetylierungsgemisch aus Acetanhydrid: Pyridin = 1:2. Nach 2 Stunden Reaktionszeit bei 40°C ist 14 vollständig in 15 übergegangen. (Produkt färbt mit Ninhydrin nicht länger rot). Es wird im Hochvakuum zur Trokkene eingeengt und der Rückstand mit Toluol/$H_2O$ aufgenommen.

Nickel und Borsalze werden durch mehrfache Extraktion mit halbgesättigter NaCl-Lösung entfernt, die Toluolphase über Natriumsulfat getrocknet, eingeengt und das Produkt an Kieselgel im System Toluol:Ethanol = 60:1 chromatographiert.

Ausbeute: 9,6 g (87%) *15*

$[\alpha]_D^{20} = -3{,}3°$ ($CHCl_3$)

Schmp.: 40°C.

Fig. 7 zeigt das $^1$H-NMR-Spektrum der Verbindung des Beispiels 15 (300 MHz, $C_6D_6$).

Fig. 8 zeigt das IR-Spektrum der Verbindung des Beispiels 15 (KBr).

Fig. 9 zeigt das Massenspektrum der Verbindung des Beispiels 15.

Beispiel 16

2-Acetamido-4,6-O-isopropyliden-1,2,5-tridesoxy-1,5-imino-D-glucit Essigsäuresalz

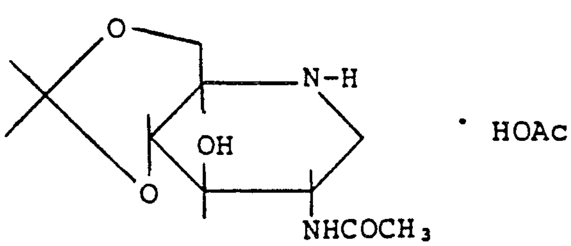

2 g (15 mmol) der Verbindung des Beispiels 15 werden in 50 ml Methanol, dem 2 ml Eisessig und 5 g Palladiumkohle 10%ig zugesetzt werden, 24 Stunden bei 3 bar hydriert. Verbindung 15 geht quantitativ in

das Essigsäure-Salz des Endproduktes über, aus dem das Endprodukt durch mehrfaches Evaporieren mit Triethylamin rein hervorgeht.

Ausbeute: 1,1 g (quantitativ)

Schmp.: des Essigsäure-Salzes: 166—169°C Zers.

$[\alpha]_D^{20}$ des Essigsäuresalzes = −4,35° (Pyridin, C=0,58).

Fig. 10 zeigt das IR-Spektrum der Verbindung des Beispiels 16 (KBr).

Fig. 11 zeigt das Massenspektrum der Verbindung des Beispiels 16.

Beispiel 17

2-Acetamido-1,2-didesoxy-nojirimycin

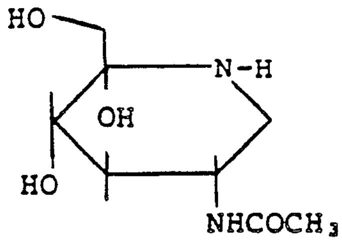

1,1 g der Verbindung des Beispiels 16 (5 mmol) werden in 20 ml eines Gemisches aus Eisessig:$H_2O$ = 3:2 gelöst und 8 Stunden auf 60°C erwärmt.

Nach Evaporation des Hydrolysegemisches, Trocknung und mehrfacher Evaporation mit Methanol/Triethylamin erhält man das Endprodukt in quantitativer Ausbeute.

$[\alpha]_D^{20}$ = + 9,9° ($H_2O$).

Schmp.: 220,5—221,7°C.

Fig. 12 zeigt das $^1$H-NMR-Spektrum der Verbindung des Beispiels 17 (300 MHz, $D_2O$).

Fig. 13 zeigt das IR-Spektrum der Verbindung des Beispiels 17 (KBr).

Fig. 14 zeigt das Massenspektrum der Verbindung des Beispiels 17.

Beispiel 18

N-Benzyl-3-O-p-methoxybenzyl-4,6-O-isopropyliden-1,5-didesoxy-1,5-imino-D-mannit

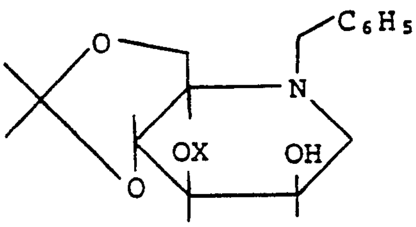

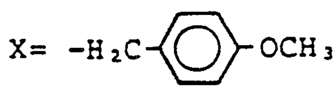

Eine Lösung von 58,6 g der Verbindung des Beispiels 10 in 700 ml abs. Toluol in der 55 g Dibutylzinnoxid suspendiert sind, wird unter $N_2$-Schutzgasatmosphäre und Anwendung eines Wasserabscheiders solange zum Rückfluß erhitzt, bis keine Wasserabscheidung mehr zu beobachten ist. Sodann wird die Temperatur auf 100°C gesenkt, und der Lösung im Stickstoffgegenstrom 6 g Tetrabutylammoniumbromid zugesetzt. Nachfolgend werden 34,5 g p-Methoxybenzylchlorid während 5 h langsam zugetropft. Nach 12 h wird dünnschichtchromatographisch (System Toluol:Aceton = 4:1) vollständige Umsetzung des Eduktes festgestellt. Die Aufarbeitung erfolgt durch Verdampfen des Lösungsmittels, Aufnehmen des Rückstandes in Chloroform und nachfolgendes Waschen der organischen Phase mit gesättigter Natriumhydrogencarbonatlösung. Dabei anfallende Zinnsalze werden durch Zentrifugation abgetrennt. Das nach Trocknen über Natriumsulfat und Verdampfung des Lösungsmittels erhaltene Rohprodukt wird säulenchromatographisch im System Toluol:Aceton = 40:1 von begleitenden Nebenprodukten befreit.

Nach Kristallisation aus Essigester/Hexan erhält man 36 g der Titelverbindung.

Fp.: 88°C.

$[\alpha]_D^{20}$ = −70,9° (Chloroform C=0,98).

Fig. 15 zeigt das $^1$H-NMR-Spektrum der Verbindung des Beispiels 18 (300 MHz, $CDCl_3$).

Fig. 16 zeigt das IR-Spektrum der Verbindung des Beispiels 18 (Nujol®).

Fig. 17 zeigt das Massenspektrum der Verbindung des Beispiels 18.

Beispiel 19

2-Acetamido-N-benzyl-1,2-didesoxy-nojirimycin

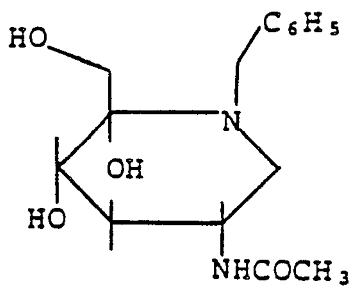

1 g (5,3 mmol) der Verbindung des Beispiels 17 werden in 20 ml Dimethylformamid gelöst, in dem 1,6 g (11,6 mmol) Kaliumcarbonat durch Rühren suspendiert werden.

Dem Gemenge werden bei 0°C 900 mg (5,3 mmol) Benzylbromid zugesetzt. Nach 8 h die Reaktion beendet. Man filtriert vom Bodenkörper ab und isoliert das Produkt nach Evaporation des Lösungsmittels in quantitativer Ausbeute.

$R_f$ = 0,3 ($CHCl_3$: MeOH = 4:1).

$[\alpha]_D^{20}$ des Essigsäuresalzes = +25,0° ($H_2O$; C = 1,41).

Fig. 18 zeigt das $^1$H-NMR Spektrum der Verbindung des Beispiels 19 (300 MHz, $D_2O$).

Fig. 19 zeigt das IR-Spektrum der Verbindung des Beispiels 19 (KBr).

Beispiel 20

2-Acetamido-N-methyl-1,2-didesoxy-nojirimycin

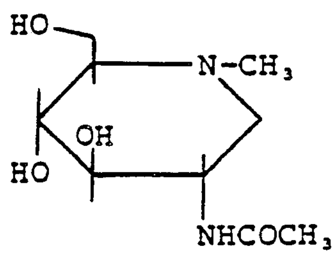

1 g (5,3 mmol) der Verbindung des Beispiels 17 werden in 30 ml einer Mischung aus Methansäure und Methanol im Verhältnis 2:1 (V/V) gelöst und 12 h auf 80°C erhitzt. Der nach Verdampfen des Lösungsmittels isolierte Rückstand wird in Methanol aufgenommen und am basischen Ionenaustauscher Typ Amberlite® CG 4001 entsaltzt.

Ausbeute: quatitativ.

$R_f$ = 0,44 (System $CHCl_3$: MeOH: $NH_3H_2O$ = 4:3:1).

$[\alpha]_D^{20}$ = + 10,72°.

Fig. 20 zeigt das $^1$H-NMR Spektrum der Verbindung des Beispiels 20 (300 MHz, $D_2O$).

Fig. 21 zeigt das IR-Spektrum der Verbindung des Beispiels 20 (KBr).

## Patentansprüche

1. 3-Amino-4,5-dihydroxypiperidine der allgemeinen Formel (I)

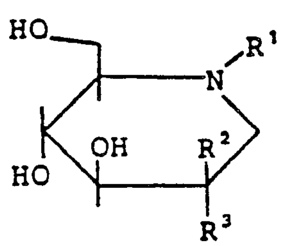

(I)

in welcher

$R^1$ für Wasserstoff, Alkyl mit bis zu 8 Kohlenstoffatomen, Aralkyl mit 7 bis 14 Kohlenstoffatomen oder für eine Gruppe der Formel

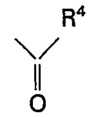

steht, wobei

$R^4$ geradkettiges oder verzweigtes Alkyl mit bis zu 8 Kohlenstoffatomen, geradkettiges oder verzweigtes Alkoxy mit bis zu 8 Kohlenstoffatomen, oder Aralkoxy mit bis zu 10 Kohlenstoffatomen bedeutet, und

$R^2$, $R^3$ für Wasserstoff oder für die Gruppe $NHR^5$ stehen, wobei

$R^5$ die gleiche Bedeutung hat wie $R^1$ und mit diesem gleich oder verschieden ist,

mit der Maßgabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

2. Verbindungen der allgemeinen Formel (I) gemäß Anspruch 1, in welcher

$R^1$ für Wasserstoff, Alkyl mit bis zu 6 Kohlenstoffatomen, für Benzyl oder für eine Gruppe der Formel

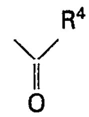

steht, worin

$R^4$ geradkettiges oder verzweigtes Alkyl mit bis zu 4 Kohlenstoffatomen, geradkettiges oder verzweigtes Alkoxy mit bis zu 4 Kohlenstoffatomen oder Benzyloxy bedeutet, und

$R^2$, $R^3$ für Wasserstoff oder für $NHR^5$ stehen, wobei

$R^5$ die gleiche Bedeutung hat wie $R^1$ und mit diesem gleich oder verschieden ist,

mit der Maßgabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

3. Verbindungen der allgemeinen Formel (I) gemäß Anspruch 1, in welcher

$R^1$ für Wasserstoff, Alkyl mit bis zu 4 Kohlenstoffatomen, Benzyl, Acetyl oder Benzyloxycarbonyl steht, und

$R^2$, $R^3$ für Wasserstoff oder für die Gruppe $NHR^5$ stehen, wobei

$R^5$ die gleiche Bedeutung hat wie $R^1$ und mit diesem gleich oder verschieden ist mit der Maßabe, daß immer ein Substituent $R^2$ oder $R^3$ für Wasserstoff steht, und der andere Substituent $R^2$ oder $R^3$ für $NHR^5$ steht, und ihre physiologisch unbedenklichen Salze.

4. Verbindungen gemäß Anspruch 1 bis 3 in der mannokonfigurierten Form (Ia)

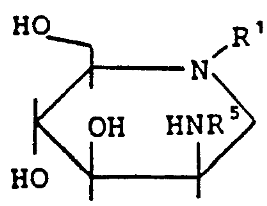

(Ia)

5. Verbindungen gemäß den Ansprüchen 1 bis 3 in der glucokonfigurierten Form (Ib)

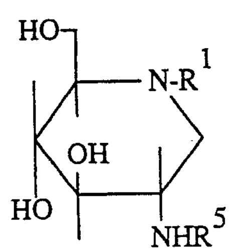

(Ib)

6. Verbindungen der allgemeinen Formel (II)

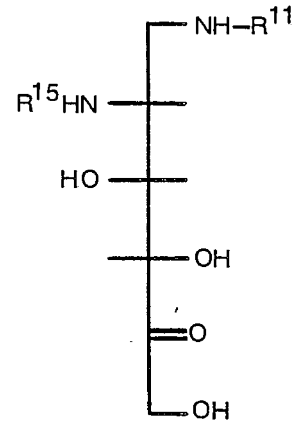

(II)

in welcher

$R^{11}$ für eine Aminoschutzgruppe, bevorzugt Benzyloxycarbonyl, Benzyl oder tert.-Butyloxycarbonyl steht und nicht Wasserstoff bedeutet,

$R^{15}$ für eine Aminoschutzgruppe ungleich $R^{11}$, bevorzugt Acetyl steht.

7. Verfahren zur Herstellung von Verbindungen der Formel (I) in der mannokonfigurierten Form

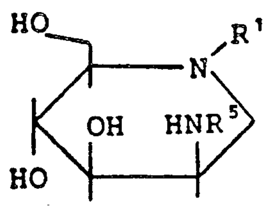

in welcher

$R^1$ für Wasserstoff, Alkyl mit bis zu 8 Kohlenstoffatomen, Aralkyl mit 7 bis 14 Kohlenstoffatomen oder für eine Gruppe der Formel

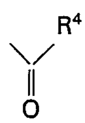

steht, wobei

$R^4$ geradkettiges oder verzweigtes Alkyl mit bis zu 8 Kohlenstoffatomen, geradkettiges oder verzweigtes Alkoxy mit bis zu 8 Kohlenstoffatomen, oder Aralkoxy mit bis zu 10 Kohlenstoffatomen bedeutet, und

$R^5$ die gleiche Bedeutung hat wie $R^1$ und mit diesem gleiche oder verschieden ist, und deren physiologisch unbedenklichen Salze, dadurch gekennzeichnet, daß man die Verbindungen der Formel (II)

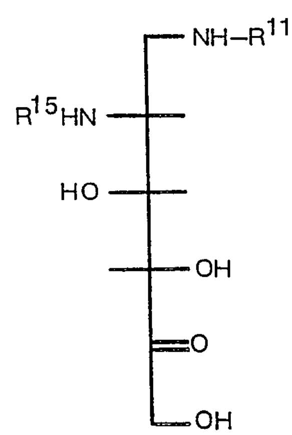

(II)

in welcher

$R^{11}$ für eine Aminoschutzgruppe, bevorzugt Benzyloxycarbonyl, Benzyl oder tert.-Butyloxycarbonyl steht und nicht Wasserstoff bedeutet, und

$R^{15}$ für eine Aminoschutzgruppe ungleich $R^{11}$, bevorzugt Acetyl steht,

in inerten Lösungsmitteln unter reduktiven Bedingungen cyclisiert, gegebenenfalls Schutzgruppen abspaltet, gegebensfalls neue Schutzgruppen einführt und gegebenenfalls anschließend mit Säure die entsprechenden Salze herstellt.

8. Verfahren zur Herstellung von Verbindungen der Formel (I), in der glucokonfigurierten Form

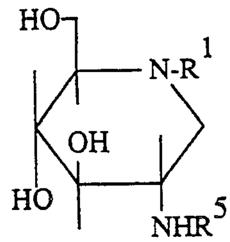

(Ib)

in welcher

$R^1$ für Wasserstoff, Alkyl mit bis zu 8 Kohlenstoffatomen, Aralkyl mit 7 bis 14 Kohlenstoffatomen oder für die Gruppe

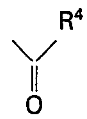

steht, worin

$R^4$ geradkettiges oder verzweigtes Alkyl mit bis zu 8 Kohlenstoffatomen, geradkettiges oder verzweigtes Alkoxy mit bis zu 8 Kohlenstoffatomen, oder Aralkoxy mit bis zu 10 Kohlenstoffatomen bedeutet, und

$R^5$ die gleiche Bedeutung hat wie $R^1$ und mit diesem gleiche oder verschieden ist, sowie ihre physiologisch unbedenklichen Salze, dadurch gekennzeichnet, daß man Azidoverbindungen der Formel (VIII)

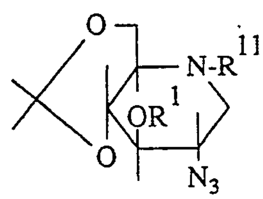

(VIII)

in welchen

$R^{11}$ für eine Aminoschutzgruppe, bevorzugt für Benzyl steht,

in inerten Lösungsmitteln in Answesenheit eines Reduktionsmittels, gegebenenfalls in Anwesenheit eines Katalysators reduziert, gegebenenfalls Schutzgruppen abspaltet, gegebenensfall Schutzgruppen in die Position $R^5$ einführt und gegebenenfalls anschließend mit Säure die entsprechenden Salze herstellt.

9. Verfahren nach Anspruch 7, dadurch gekennzeichnet, daß man die Cyclisierung mit Wasserstoff bei einem Wasserstoffdruck von 1 bis 150 bar in Gegenwart von Pd oder Pt in Wasser, Alkohol, Ether, DMF oder Eisessig durchführt.

10. Verfahren nach Anspruch 9, dadurch gekennzeichnet, daß man die Cyclisierung bei Temperaturen von 20 bis 150°C durchführt.

11. Verfahren nach Anspruch 7, dadurch gekennzeichnet, daß man die Cyclisierung in Gegenwart von Alkalimetallcyanborhydriden, Dialkylaminoboranen oder Alkalimetallborhydriden durchgeführt.

12. Verfahren nach Anspruch 11, dadurch gekennzeichnet, daß man die Cyclisierung bei Normaldruck und Temperaturen von 0°C bis 150°C durchführt.

13. Arzneimittel, enthaltend 3-Amino-4,5-dihydroxy-piperidine gemäß Anspruch 1.

14. Verwendung von 3-Amino-4,5-dihydroxy-piperidinen gemäß Anspruch 1 zur Herstellung von Arzneimitteln.

## Revendications

1. 3-amino-4,5-dihydroxypipéridines de formule générale (I)

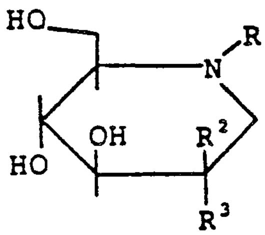

(I)

dans laquelle

$R^1$ représente un atome d'hydrogène, un groupe alkyle contenant jusqu'à 8 atomes de carbone, un groupe aralkyle contenant de 7 à 14 atomes de carbone ou un groupe de formule

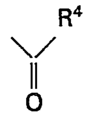

dans laquelle

$R^4$ représente un groupe alkyle à chaîne droite ou ramifiée, contenant jusqu'à 8 atomes de carbone, un groupe alcoxy à chaîne droite ou ramifiée, contenant jusqu'à 8 atomes de carbone, ou un groupe arylcoxy contenant jusqu'à 10 atomes de carbone, tandis que

$R^2$, $R^3$ représentent un atome d'hydrogène ou le groupe $NHR^5$,

dans lequel

$R^5$ a la même signification que $R^1$ et lui est identique ou différent,

avec cette réserve qu'un substituant $R^2$ ou $R^3$ représente toujours un atome d'hydrogène, tandis que l'autre substituant $R^2$ ou $R^3$ représente $NHR^5$, ainsi que leurs sels physiologiquement acceptables.

2. Composés de formule générale (I), selon la revendication 1, formule dans laquelle

$R^1$ représente un atome d'hydrogène, un groupe alkyle contenant jusqu'à 6 atomes de carbone, un groupe benzyle ou un groupe de formule

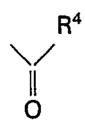

dans laquelle

$R^4$ représente un groupe alkyle à chaîne droite ou ramifiée, contenant jusqu'à 4 atomes de carbone, un groupe alcoxy à chaîne droite ou ramifiée, contenant jusqu'à 4 atomes de carbone, ou un groupe benzyloxy, tandis que

$R^2$, $R^3$ représentent un atome d'hydrogène ou $NHR^5$,

dans lequel

$R^5$ a la même signification que $R^1$ et lui est identique ou différent,

avec cette réserve qu'un substituant $R^2$ ou $R^3$ représente toujours un atome d'hydrogène, tandis que l'autre substituant $R^2$ ou $R^3$ représente $NHR^5$, ainsi que leurs sels physiologiquement acceptables.

3. Composés de formule générale (I), selon la revendication 1, formule dans laquelle

$R^1$ représente un atome d'hydrogène, un groupe alkyle contenant jusqu'à 4 atomes de carbone, un groupe benzyle, un groupe acétyle, ou un groupe benzyloxycarbonyle tandis que

$R^2$, $R^3$ représentent un atome d'hydrogène ou le groupe $NHR^5$,

dans lequel

$R^5$ a la même signification que $R^1$, tandis qu'il lui est identique ou différent,

avec cette réserve qu'un substituant $R^2$ ou $R^3$ représente toujours un atome d'hydrogène, tandis que l'autre substituant $R^2$ ou $R^3$ représente $NHR^5$,

ainsi que leurs sels physiologiquement acceptables.

4. Composés selon la revendication 1 à 3, de forme (Ia) à configuration manno

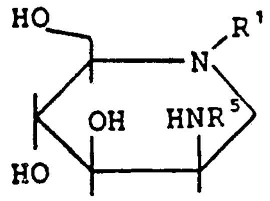

(Ia)

5. Composés selon les revendications 1 à 3, de forme (Ib) à configuration gluco

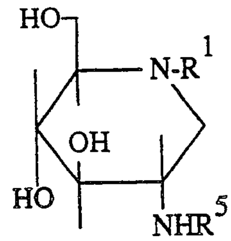

(Ib)

6. Composés de formule générale (II)

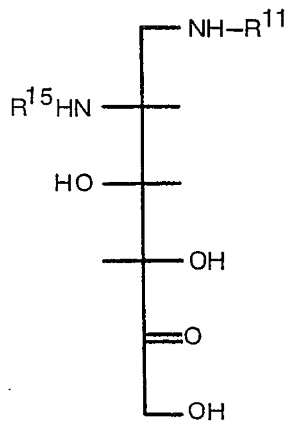

(II)

dans laquelle

$R^{11}$ représente un groupe protecteur du groupe amino, de préférence, un groupe benzyloxycarbonyle, un groupe benzyle, ou un groupe tert.-butyloxycarbonyle, tandis qu'il ne représente pas un atome d'hydrogène,

$R^{15}$ représente un groupe protecteur du groupe amino différent de $R^{11}$, de préférence, un groupe acétyle.

7. Procédé de préparation des composés de formule (I) de forme à configuration manno

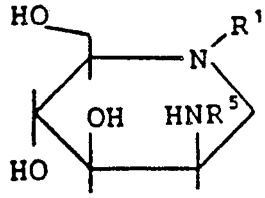

(Ia)

dans laquelle

$R^1$ représente un atome d'hydrogène, un groupe alkyle contenant jusqu'à 8 atomes de carbone, un groupe aralkyle contenant de 7 à 14 atomes de carbone, ou le groupe

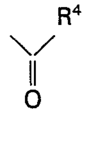

dans lequel

$R^4$ représente un groupe alkyle à chaîne droite ou ramifiée, contenant jusqu'à 8 atomes de carbone, un groupe alcoxy à chaîne droite ou ramifiée, contenant jusqu'à 8 atomes de carbone, ou un groupe aralcoxy contenant jusqu'à 10 atomes de carbone, tandis que

$R^5$ a la même signification que $R^1$, tandis qu'il lui est identique ou différent,

ainsi que leurs sels physiologiquement acceptables, caractérisé en ce que l'on cyclise les composés de formule (II)

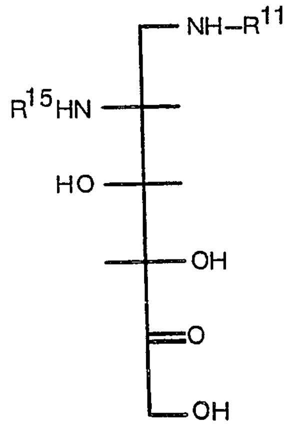

(II)

dans laquelle

$R^{11}$ représente un groupe protecteur du groupe amino, de préférence, un groupe benzyloxycarbonyle, un groupe benzyle, ou un groupe tert.-butyloxycarbonyle, tandis qu'il ne représente pas un atome d'hydrogène,

et

$R^{15}$ représente un groupe protecteur du groupe amino différent de $R^{11}$, de préférence, un groupe acétyle,

dans des solvants inertes et dans des conditions de réduction, en ce que l'on élimine éventuellement les groupes producteurs, que l'on introduit éventuellement d'autres groupes protecteurs et qu'avec de l'acide, on prépare ensuite éventuellement les sels.correspondants.

8. Procédé de préparation des composés de formule (I) de forme à configuration gluco

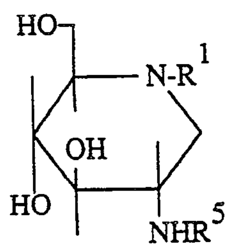

(Ib)

dans laquelle

$R^1$ représente un atome d'hydrogène, un groupe alkyle contenant jusqu'à 8 atomes de carbone, un groupe aralkyle contenant de 7 à 14 atomes de carbone, ou le groupe

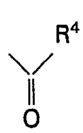

dans lequel

$R^4$ représente un groupe alkyle à chaîne droite ou ramifiée, contenant jusqu'à 8 atomes de carbone, un groupe alcoxy à chaîne droite ou ramifiée, contenant jusqu'à 8 atomes de carbone, ou un groupe aralcoxy contenant jusqu'à 10 atomes de carbone, tandis que

$R^5$ a la même signification que $R^1$, tandis qu'il lui est identique ou différent,

ainsi que leurs sels physiologiquement acceptables, caractérisé en ce que l'on réduit les composés azido de formule (VIII)

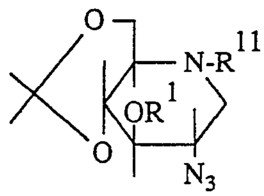

(VIII)

dans laquelle

$R^{11}$ représente un groupe protecteur du groupe amino, de préférence, un groupe benzyle,

dans des solvants inertes, en présence d'un agent de réduction, éventuellement en présence d'un catalyseur, en ce qu'on l'on élimine éventuellement les groupes protecteurs, en ce que l'on introduit éventuellement de nouveaux groupes protecteurs en position $R^5$, et en ce qu'éventuellement, avec de l'acide, on prépare enfin les sels correspondants.

9. Procédé selon la revendication 7, caractérisé en ce que l'on effectue la cyclisation avec de l'hydrogène, sous une pression d'hydrogène de 1 à 150 bars, en présence de Pd ou de Pt dans de l'eau, de l'alcool, de l'éther, du diméthylformamide ou de l'acide acétique glacial.

10. Procédé selon la revendication 9, caractérisé en ce que l'on effectue la cyclisation à des températures allant de 20 à 150°C.

11. Procédé selon la revendication 7, caractérisé en ce que l'on effectue la cyclisation en présence de cyanoborohydrures de métaux alcalins, de dialkylaminoboranes ou de borohydrures de métaux alcalins.

12. Procédé selon la revendication 11, caractérisé en ce que l'on effectue la cyclisation sous pression normale et à des températures allant de 0°C à 150°C.

13. Médicament contenant des 3-amino-4,5-dihdroxypipéridines selon la revendication 1.

14. Utilisation de 3-amino-4,5-dihydroxypipéridines selon la revendication 1, pour la préparation de médicaments.

**Claims**

1. 3-Amino-4,5-dihydroxypiperidines of the general formula (I)

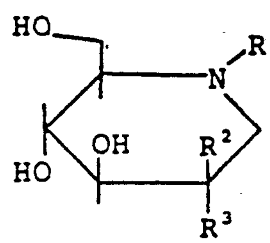

(I)

in which

$R^1$ represents hydrogen, alkyl having up to 8 carbon atoms, aralkyl having 7 to 14 carbon atoms, or represents a group of the formula

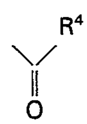

$R^4$ denoting straight-chain or branched alkyl having up to 8 carbon atoms, straight-chain or branched alkoxy having up to 8 carbon atoms, or aralkoxy having up to 10 carbon atoms, and

$R^2$ and $R^3$ represent hydrogen or represent the group $NHR^5$,

$R^5$ having the same meaning as $R^1$ and being identical to or different from the latter,

with the proviso that, in every case, one substituent $R^2$ or $R^3$ represents hydrogen and the other substituent $R^2$ or$R^3$ represents $NHR^5$, and their physilogically acceptable salts.

2. Compounds of the general formula (I) according to Claim 1, in which

$R^1$ represents hydrogen or alkyl having up to 6 carbon atoms, represents benzyl or represents a group of the formula

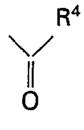

in which

$R^4$ denotes straight-chain or branched alkyl having up to 4 carbon atoms, straight-chain or branched

alkoxy having up to 4 carbon atoms, or benzyloxy, and

$R^2$ and $R^3$ represent hydrogen or represent $NHR^5$, $R^5$ having the same meaning as $R^1$ and being identifical to or different from the latter,

with the proviso that, in every case, one substituent $R^2$ or $R^3$ represents hydrogen and the other substituent $R^2$ or $R^3$ represents $NHR^5$, and their physiologically acceptable salts.

3. Compounds of the general formula (I) according to Claim 1, in which

$R^1$ represents hydrogen, alkyl having up to 4 carbon atoms, benzyl, acetyl or benzyloxycarbonyl, and

$R^2$ and $R^3$ represent hydrogen or represent the group $NHR^5$,

$R^5$ having the same meaning as $R^1$ and being identical to or different from the latter,

with the proviso that, in every case, one substituent $R^2$ or $R^3$ represents hydrogen and the other substituent $R^2$ or $R^3$ represents $NHR^5$, and their physiologically acceptable salts.

4. Compounds according to Claim 1 to 3 in the form having the manno configuration (Ia)

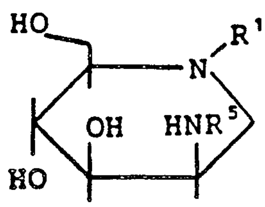

(Ia)

5. Compounds according to Claims 1 to 3 in the form having the gluco configuration (Ib)

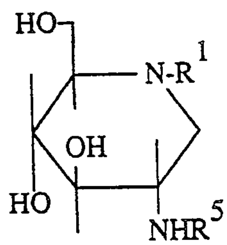

(Ib)

6. Compounds of the general formula (II)

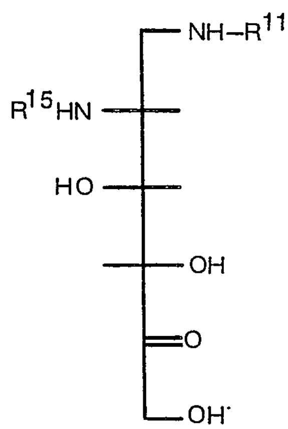

(II)

in which

$R^{11}$ represents an amino protective group, preferably benzyloxycarbonyl, benzyl or tert.-butyloxycarbonyl and does not denote hydrogen,

$R^{15}$ represents an amino protective group different from $R^{11}$, preferably acetyl.

7. Process for the preparation of compounds of the formula (I) in the form having the manno configuration

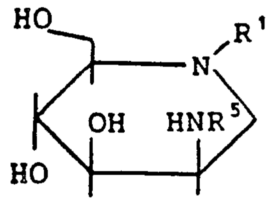

in which

$R^1$ represents hydrogen, alkyl having up to 8 carbon atoms, aralkyl having 7 to 14 carbon atoms, or represents a group of the formula

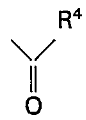

$R^4$ denoting straight-chain or branched alkyl having up to 8 carbon atoms, straight-chain or branched alkoxy having up to 8 carbon atoms, or aralkoxy having up to 10 carbon atoms, and

$R^5$ has the same meaning as $R^1$ and is identical to or different from the latter,

and their physiologically acceptable salts, characterised in that the compounds of the formula (II)

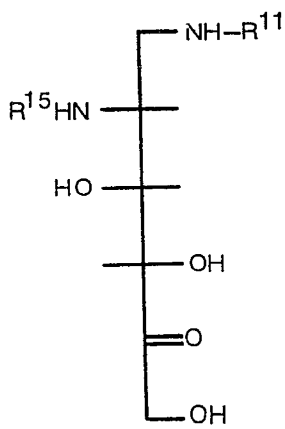

(II)

in which

$R^{11}$ represents an amino protective group, preferably benzyloxycarbonyl, benzyl or tert.-butyloxycarbonyl, and does not denote hydrogen, and

$R^{15}$ represents an amino protective group different from $R^{11}$, preferably acetyl,

are cyclized in inert solvents under reductive conditions, where appropriate protective groups are eliminated, where appropriate new protective groups are introduced, and where appropriate then the corresponding salts are prepared with acid.

8. Process for the preparation of compounds of the formula (I) in the form having the gluco configuration

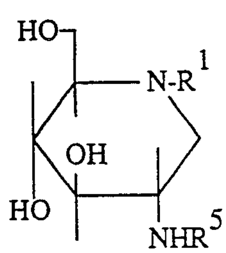

(Ib)

in which

$R^1$ represents hydrogen, alkyl having up to 8 carbon atoms, aralkyl having 7 to 14 carbon atoms, or represents the group

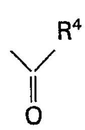

in which $R^4$ denotes straight-chain or branched alkyl having up to 8 carbon atoms, straight-chain or branched alkoxy having up to 8 carbon atoms, or aralkoxy having up to 10 carbon atoms, and

$R^5$ has the same meaning as $R^1$ and is identical to or different from the latter,

and their physiologically acceptable salts, characterized in that azido compounds of the formula (VIII)

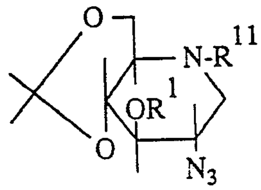

(VIII)

in which

$R^{11}$ represents an amino protective group, preferably benzyl

are reduced in inert solvents in the presence of a reducing agent and where appropriate in the presence of a catalyst, where appropriate protective groups are eliminated, where appropriate protective groups are introduced into the $R^5$ position and where appropriate the corresponding salts are then prepared with acid.

9. Process according to Claim 7, characterized in that the cyclization is carried out with hydrogen under a hydrogen pressure of 1 to 50 bar in the presence of Pd or Pt in water, alcohol, ether, DMF or glacial acetic acid.

10. Process according to Claim 9, characterized in that the cyclization is carried out at temperatures of 20 to 150°C.

11. Process according to Claim 7, characterized in that the cyclization is carried out in the presence of alkali metal cyanoborohydrides, dialkylaminoboranes or alkali metal borohydrides.

12. Process according to Claim 11, characterized in that the cyclization is carried out under normal pressure and at temperatures of 0°C to 150°C.

13. Medicaments containing 3-amino-4,5-dihydroxy-piperidines according to Claim 1.

14. Use of 3-amino-4,5-dihydroxy-piperidines according to Claim 1 for the preparation of medicaments.

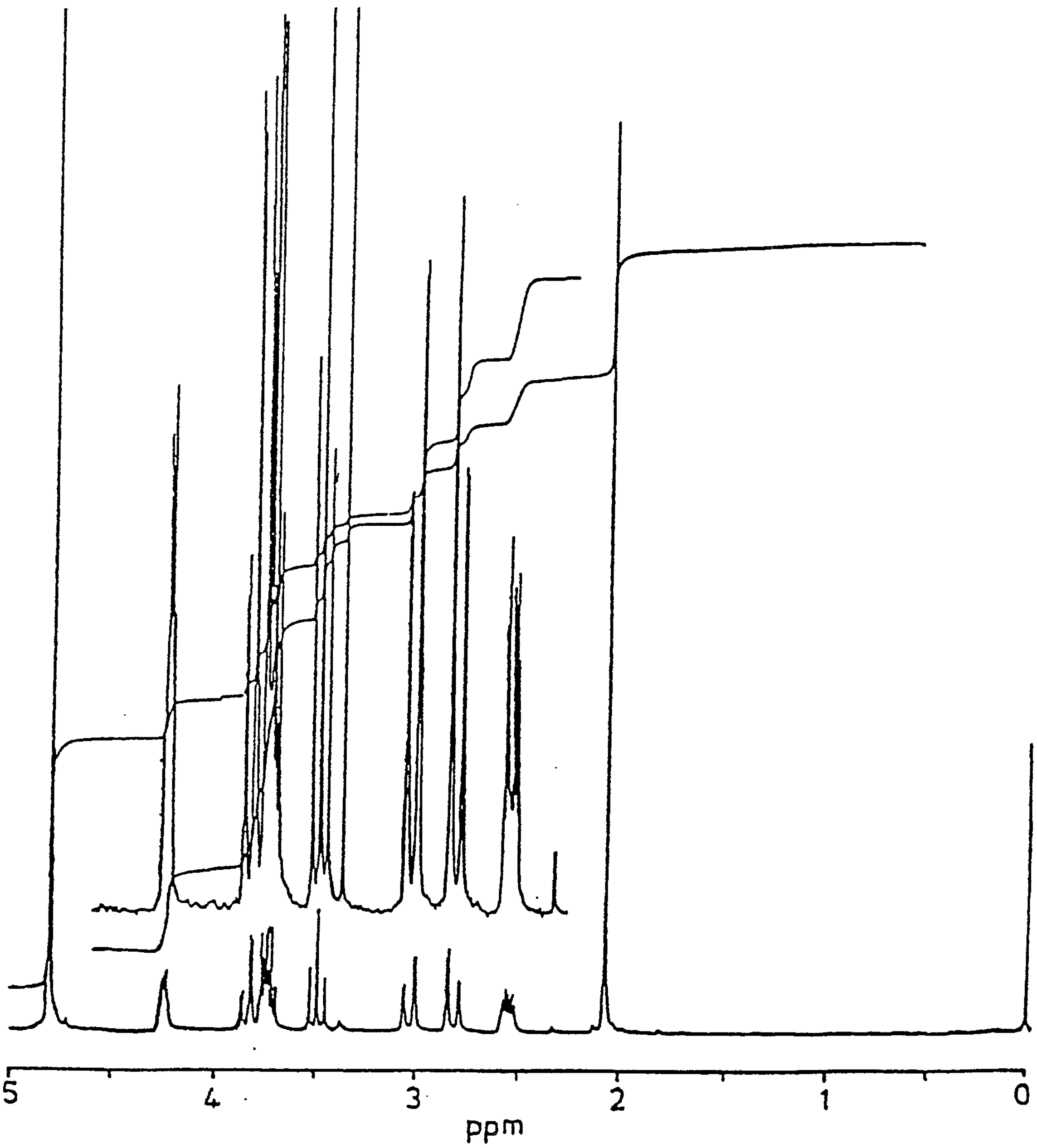


FIG. 1

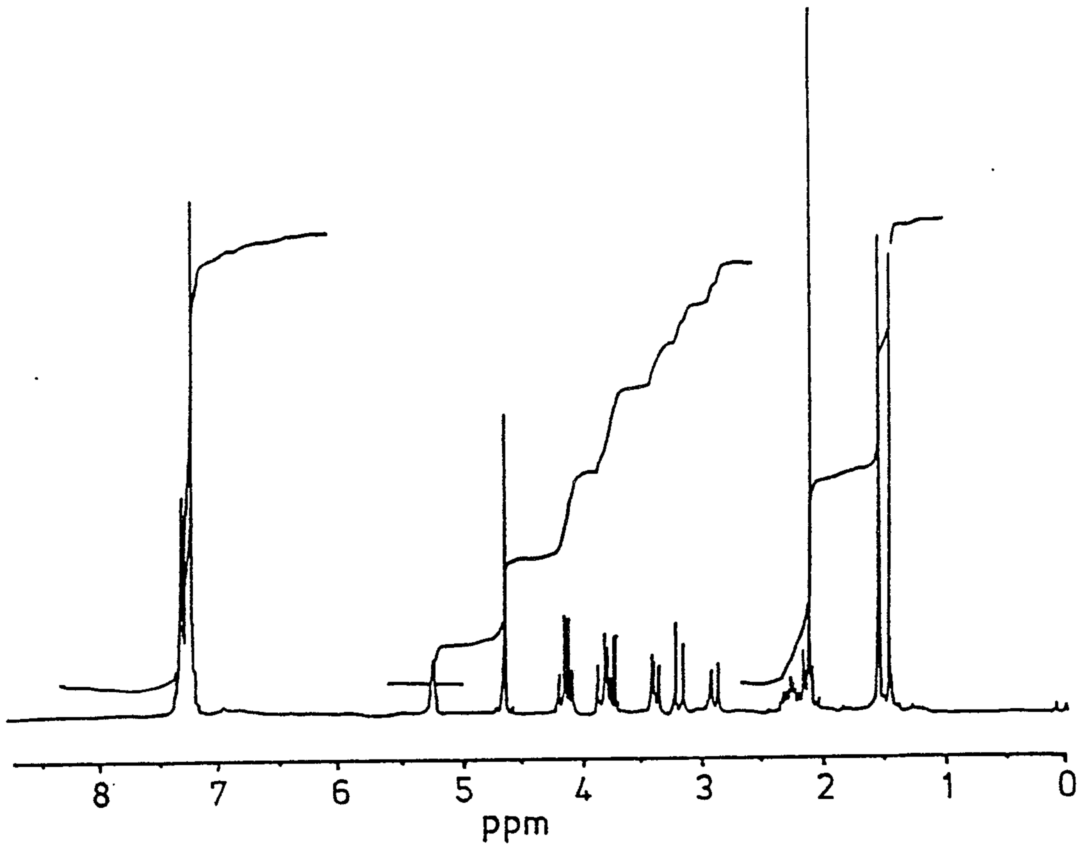


FIG. 2

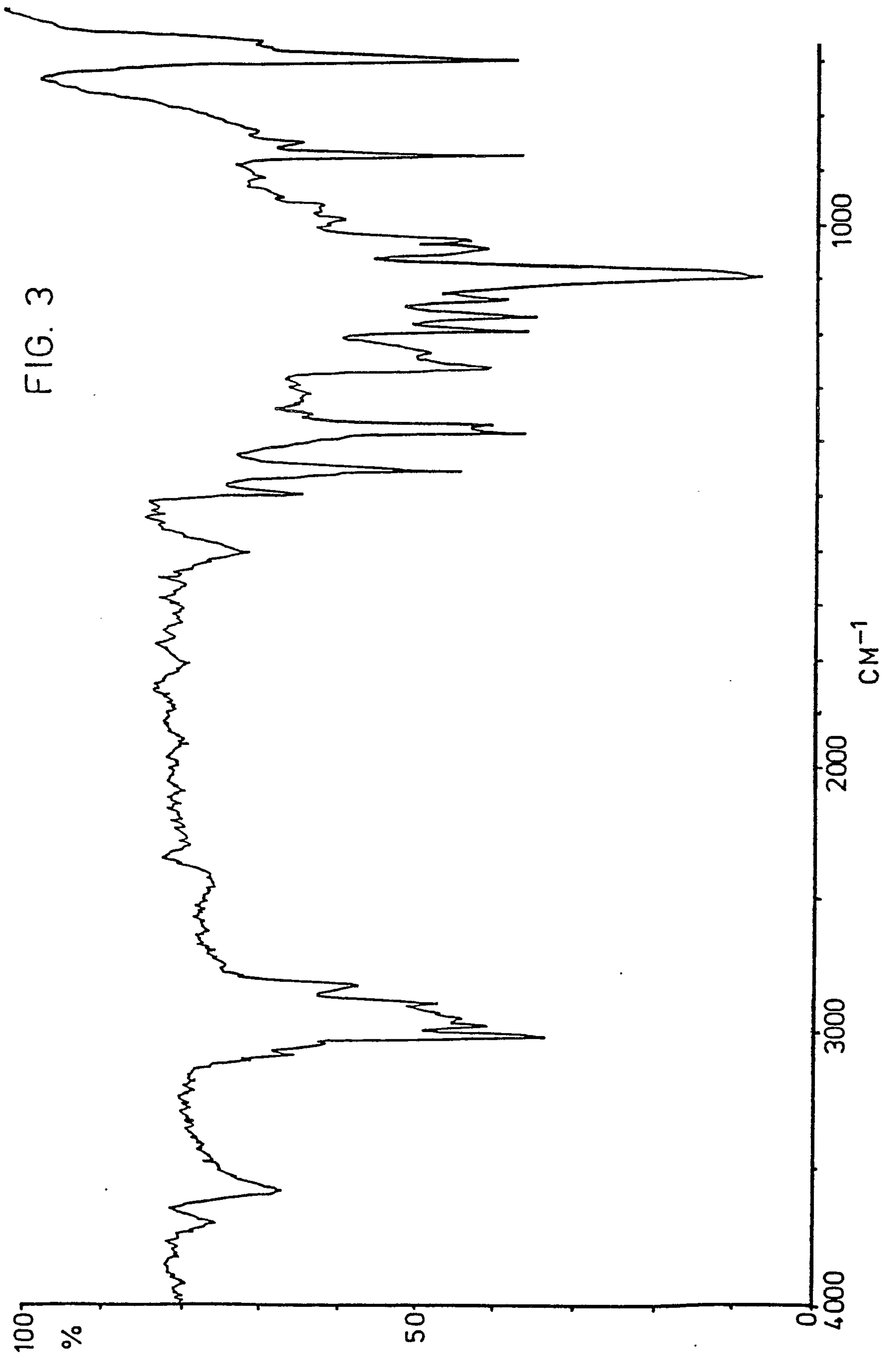


FIG. 3

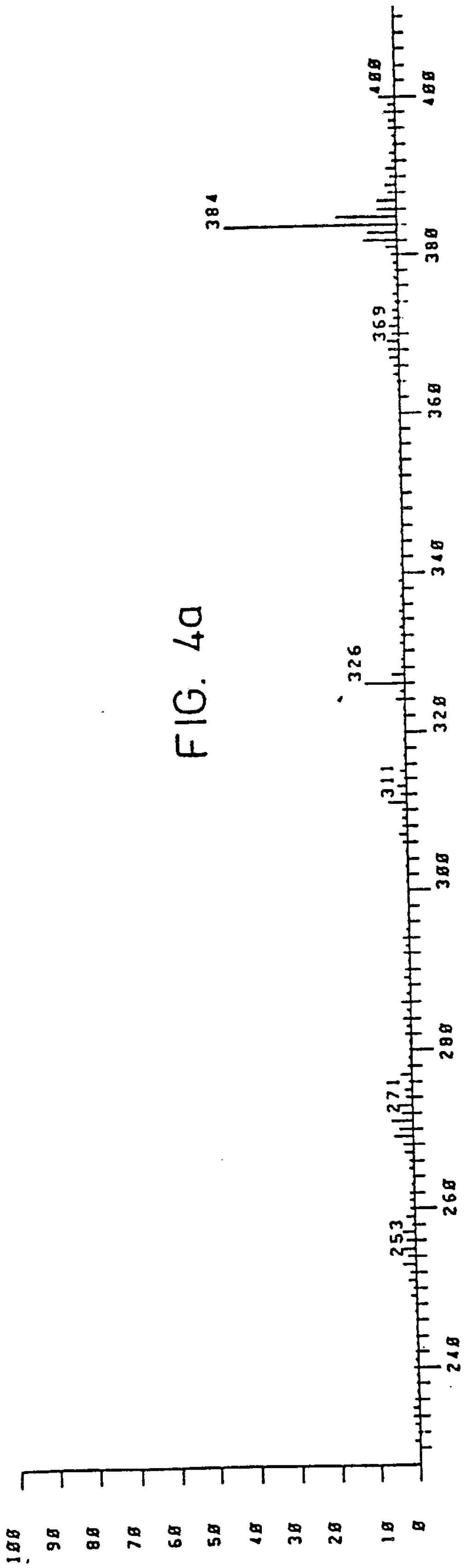
384
369
326
311
271
253
240
260
280
300
320
340
360
380
400
100
90
80
70
60
50
40
30
20
10
0

FIG. 4a

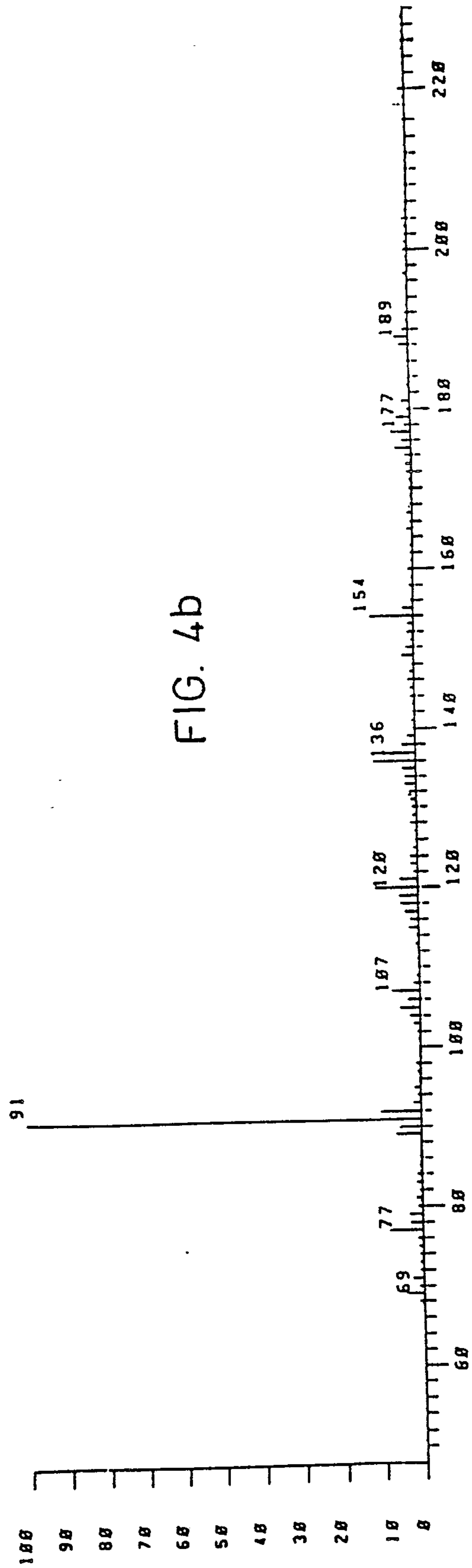
91
189
177
154
136
120
107
77
69
60
80
100
120
140
160
180
200
220
100
90
80
70
60
50
40
30
20
10
0

FIG. 4b

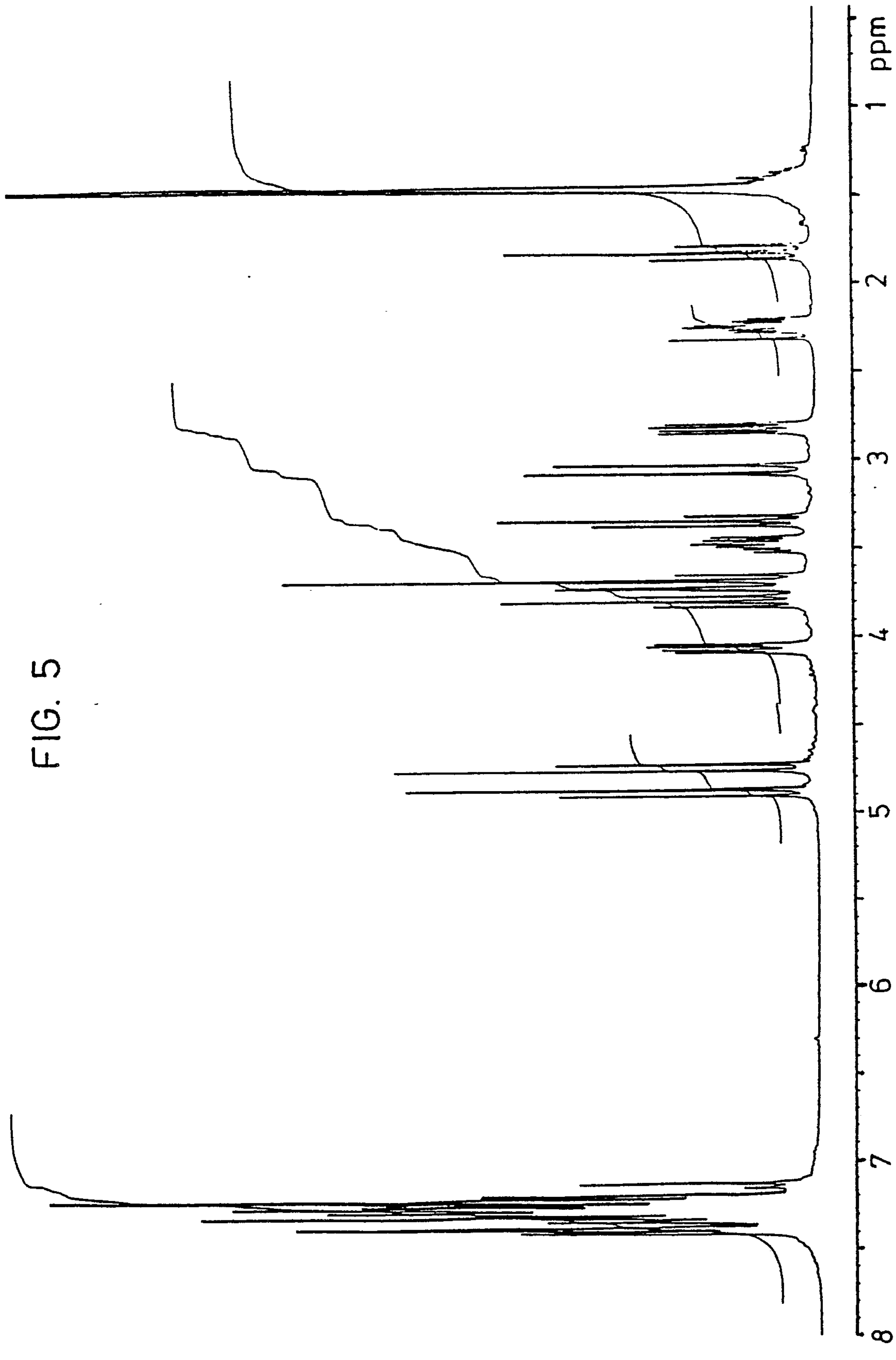


FIG. 5

FIG. 6

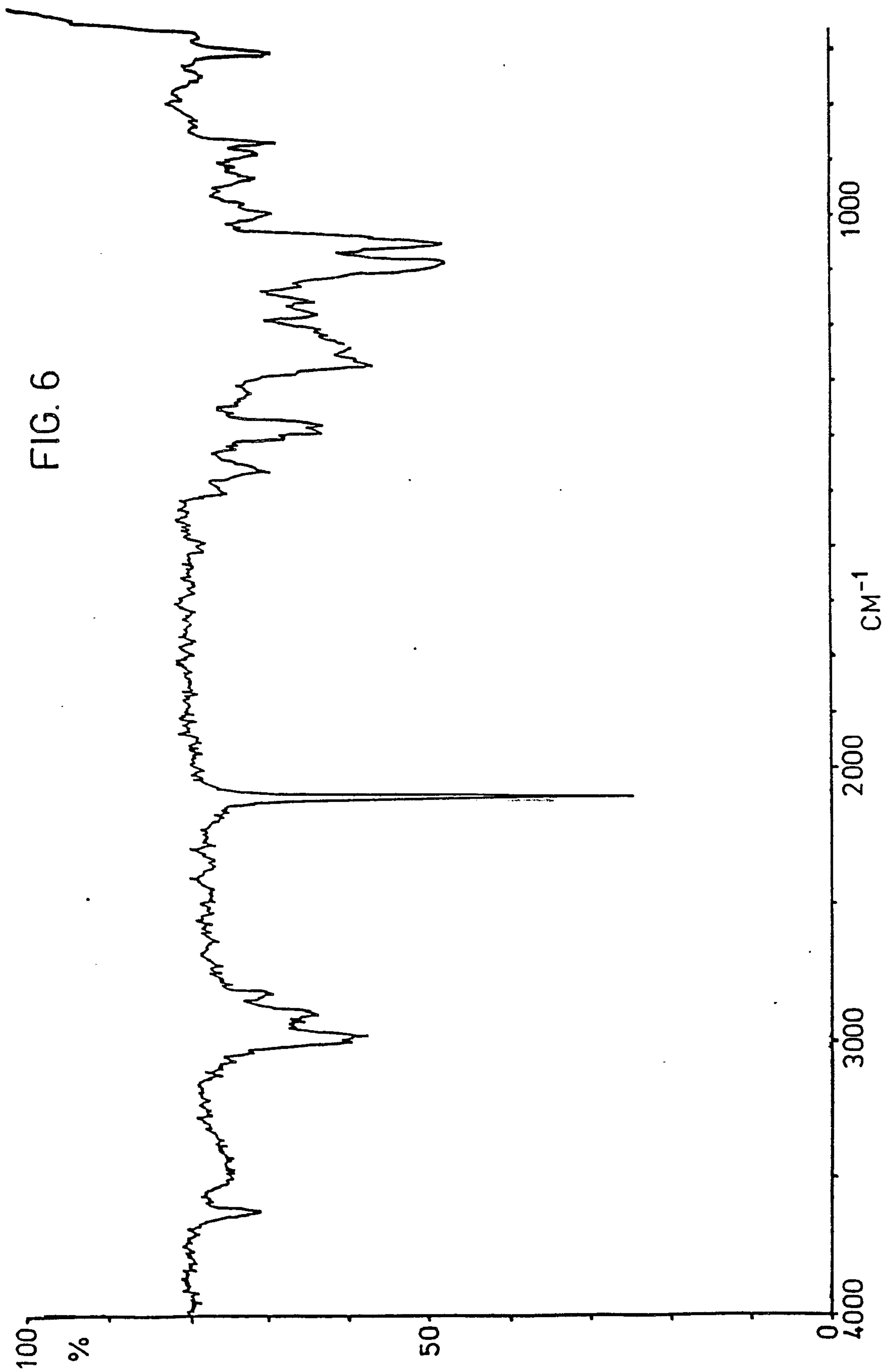

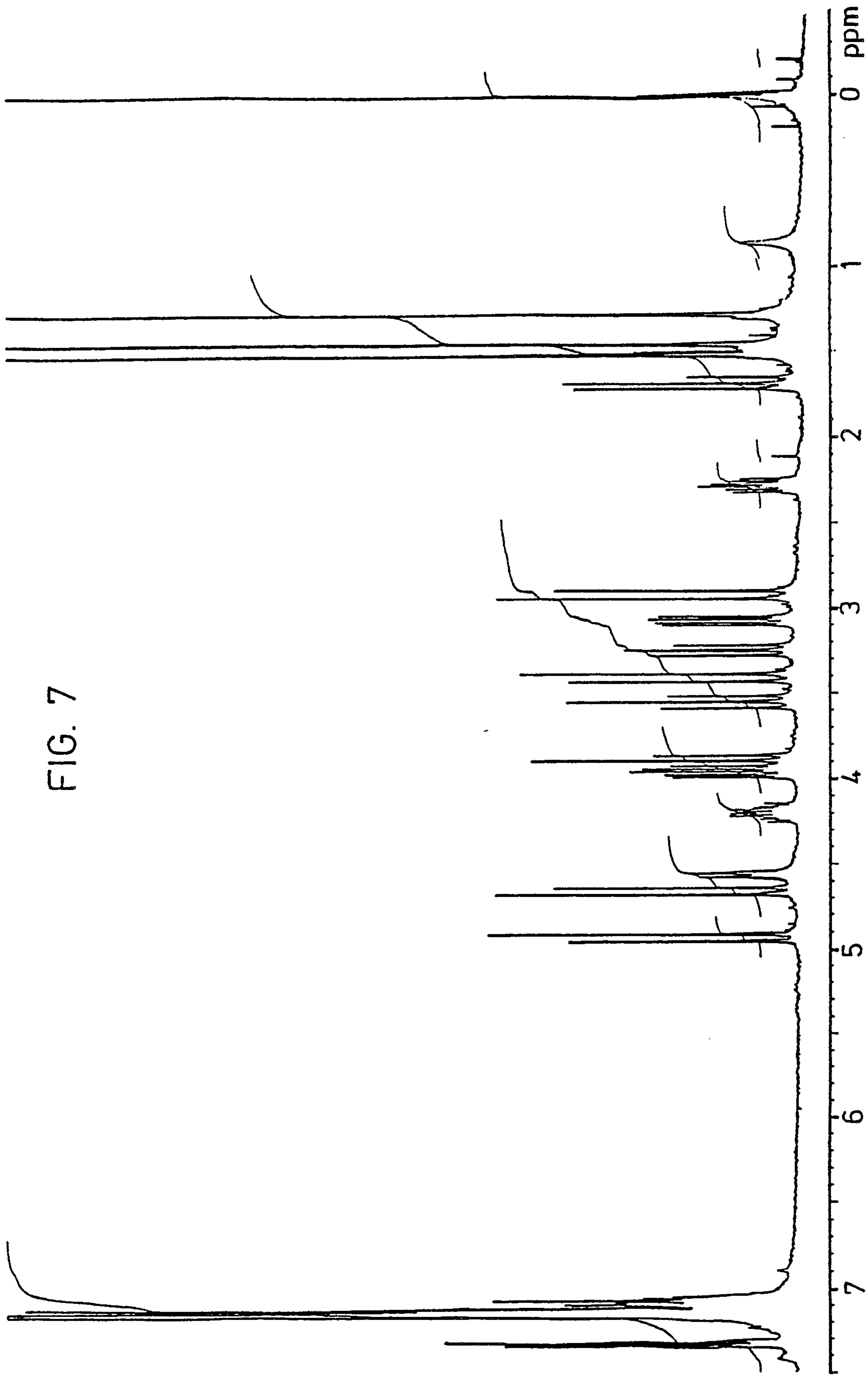


FIG. 7

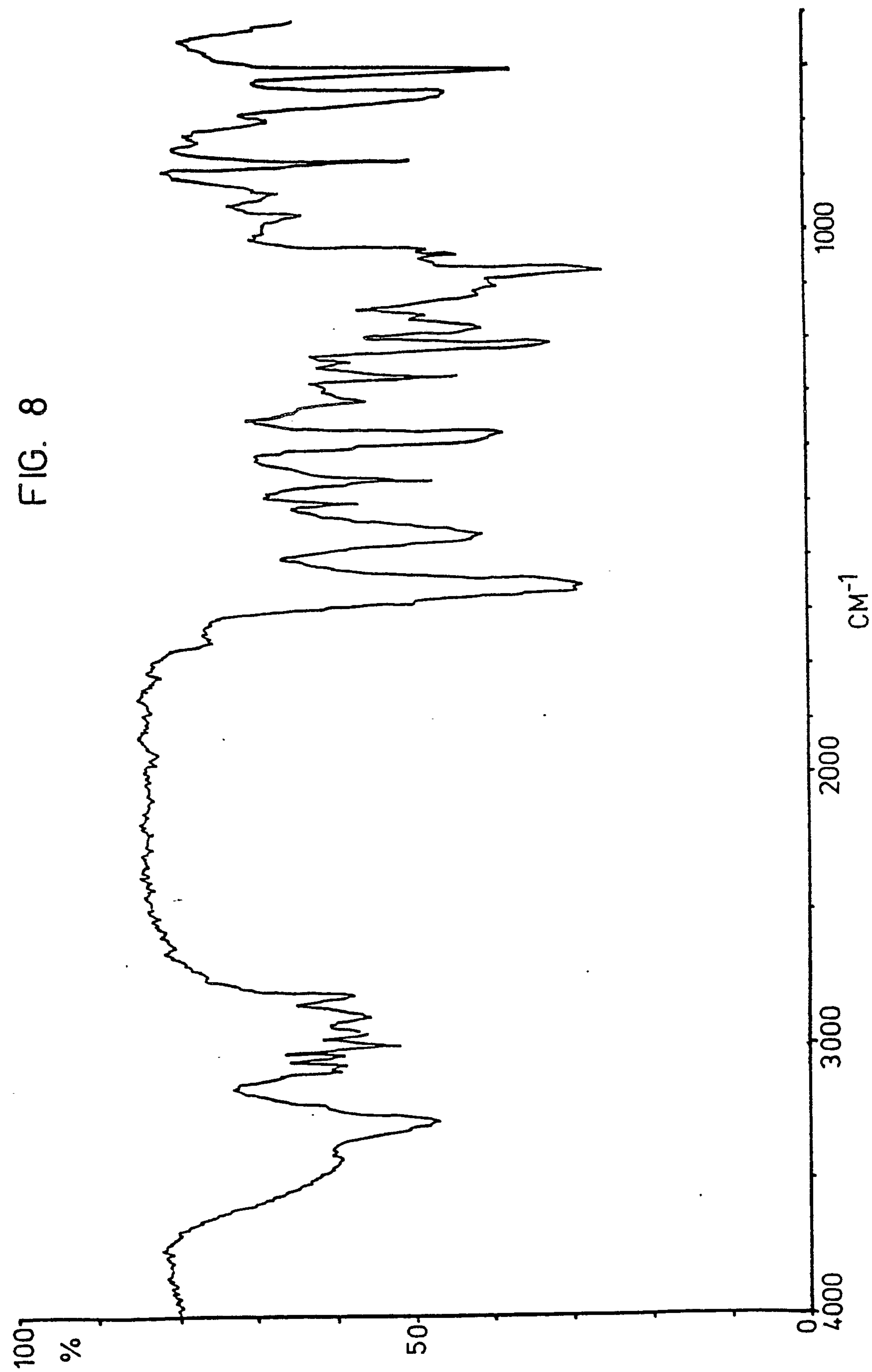


FIG. 8

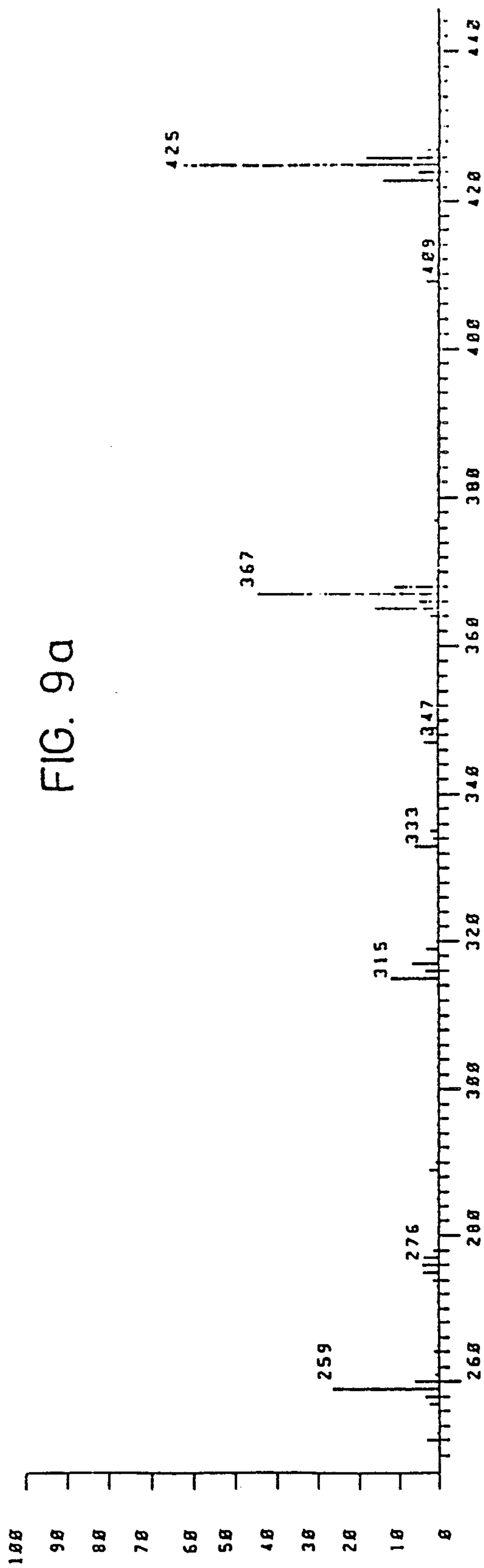


FIG. 9a

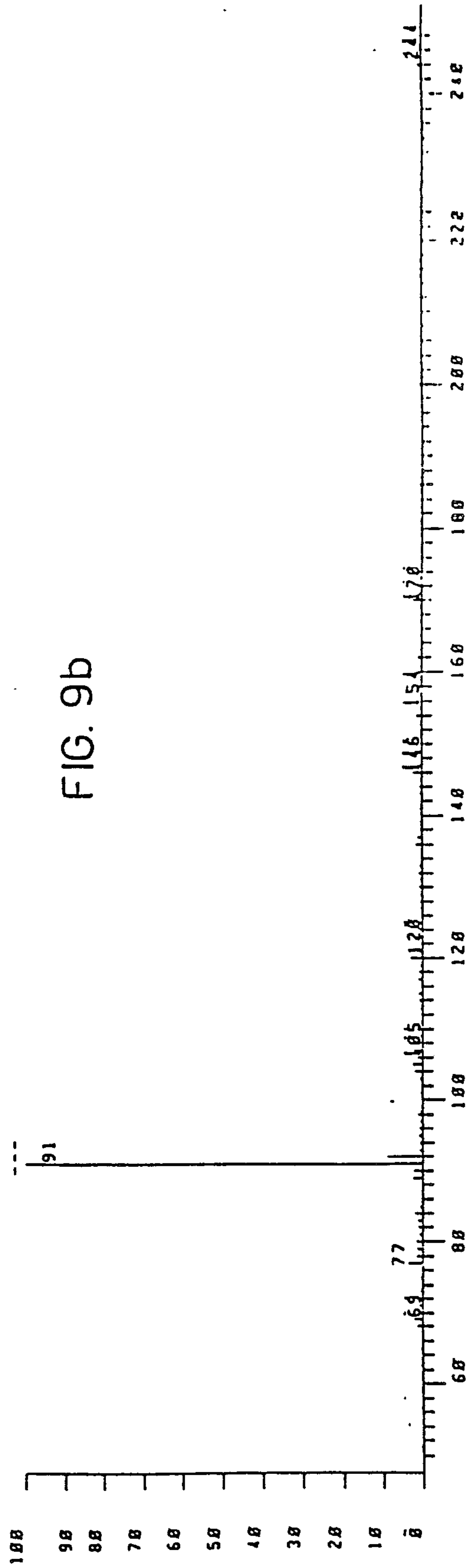


FIG. 9b

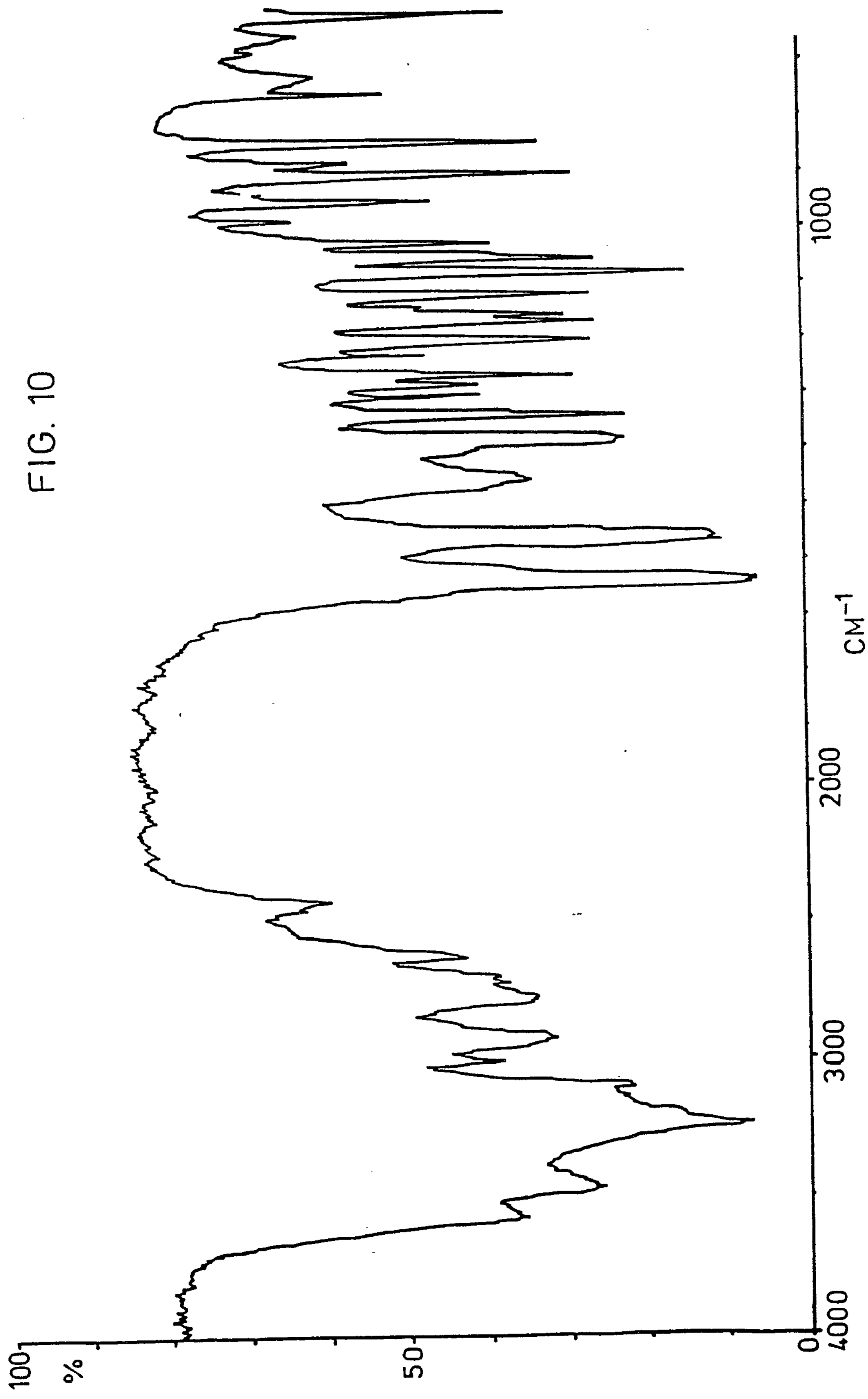


FIG. 10

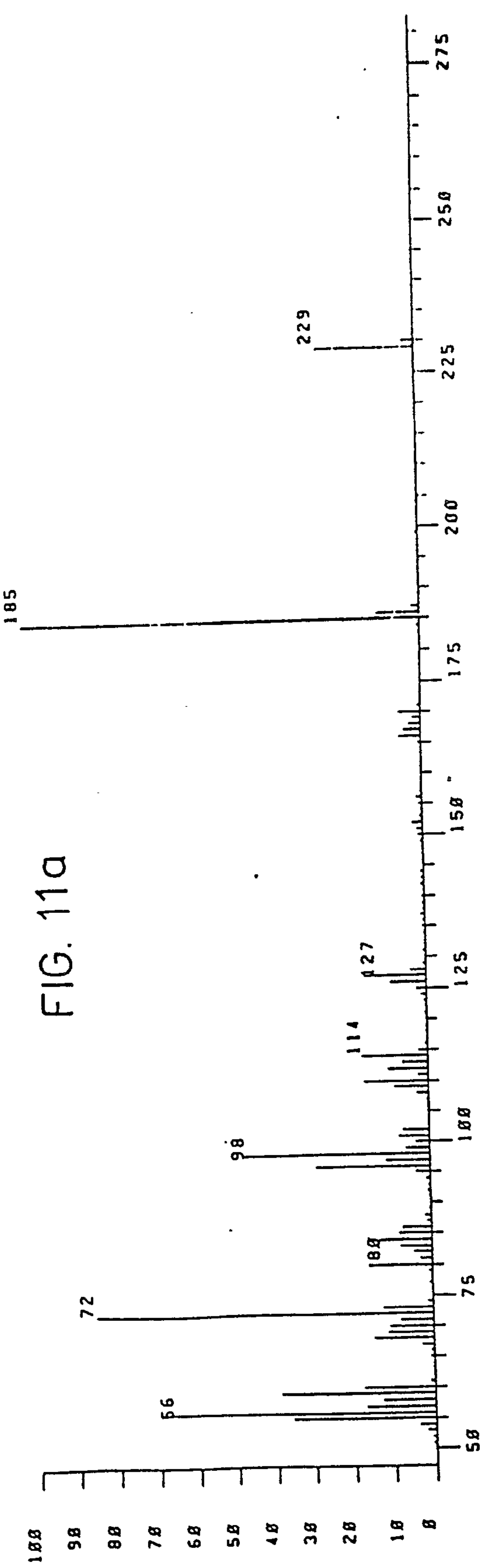


FIG. 11a

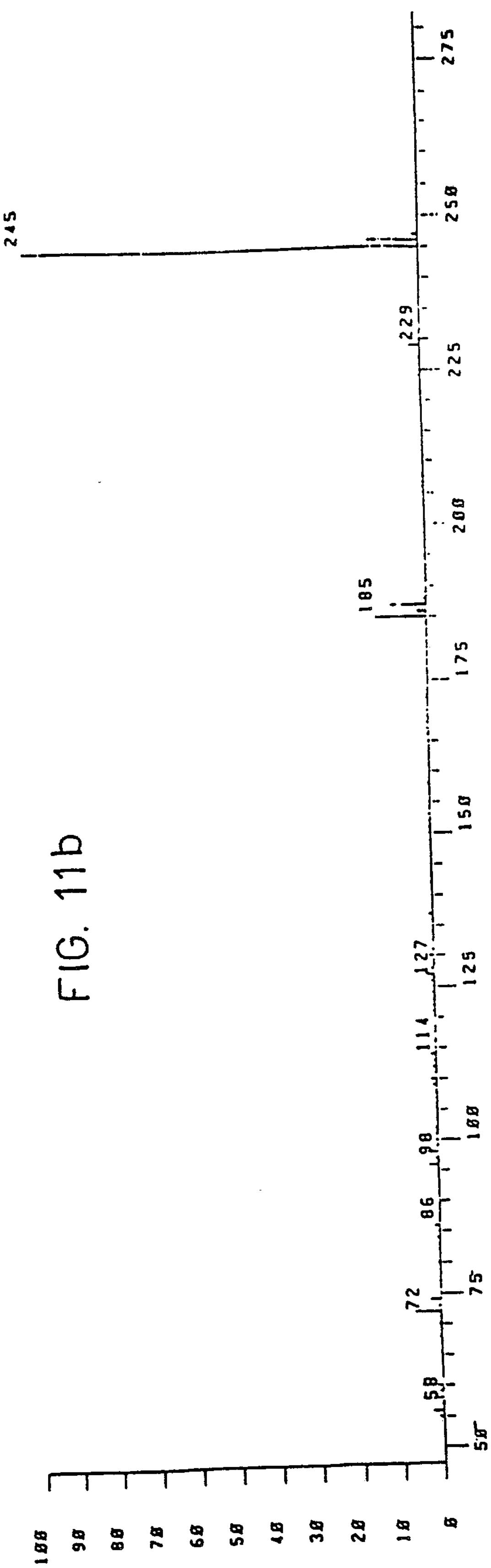


FIG. 11b

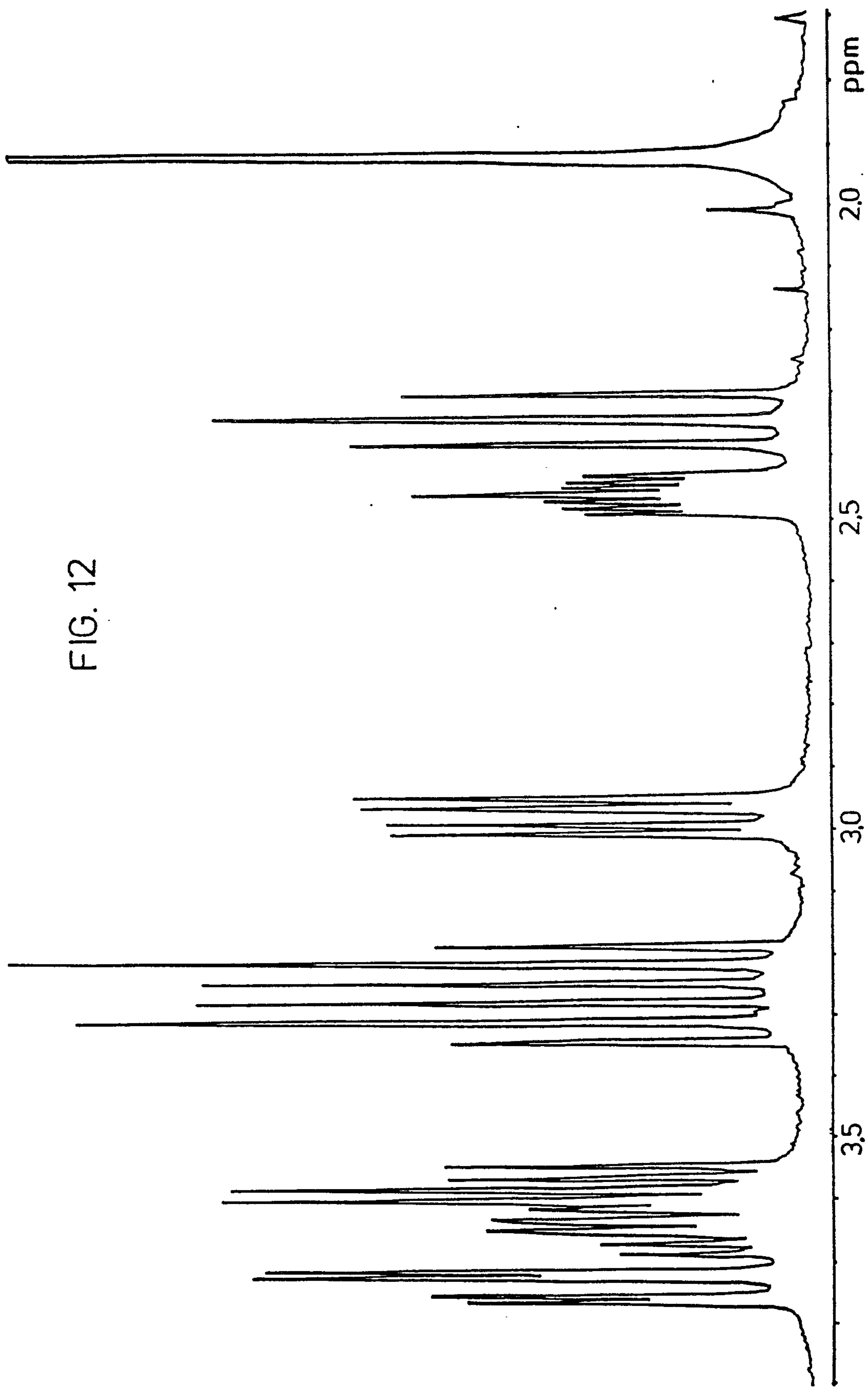


FIG. 12

FIG. 13

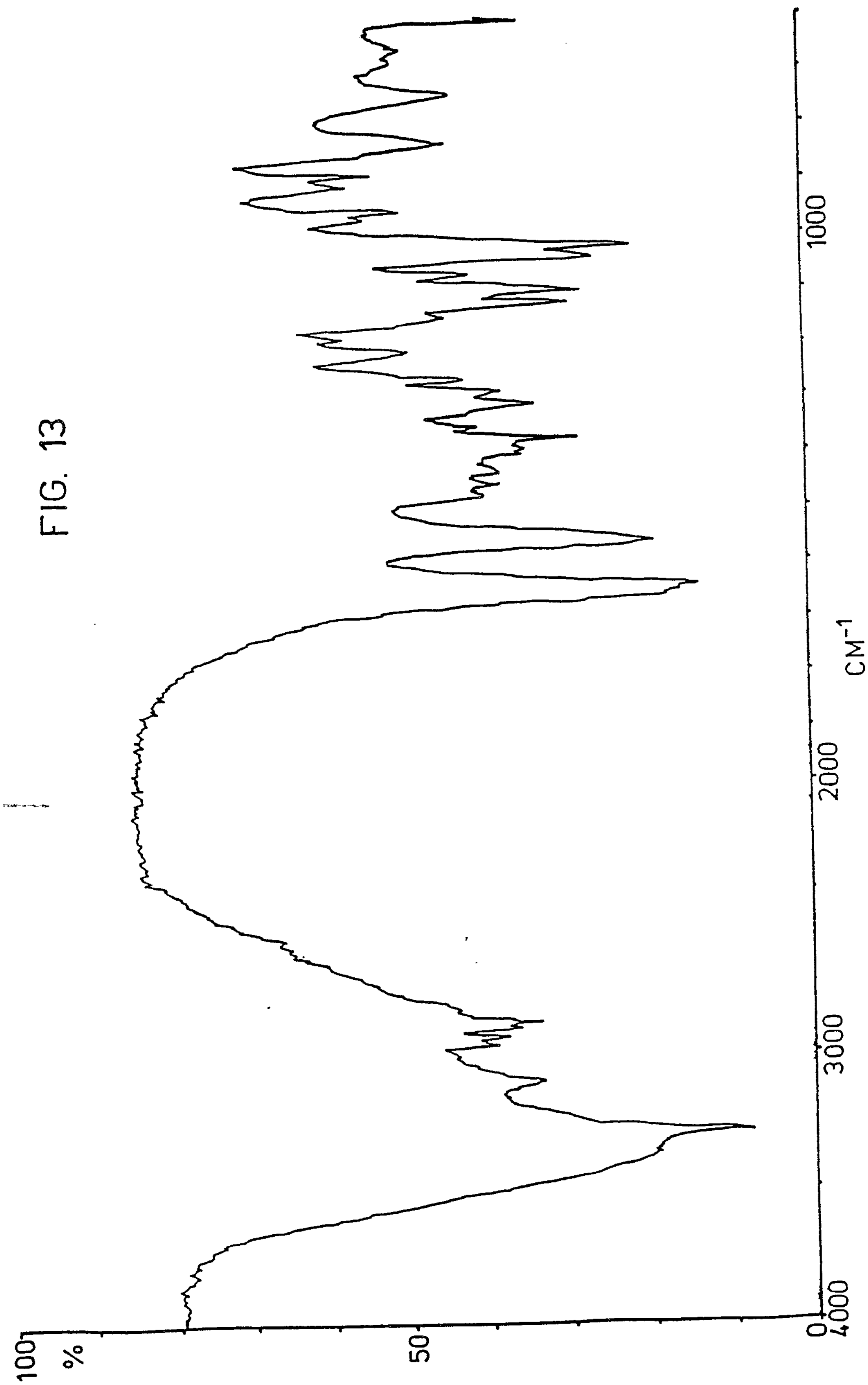

FIG. 14 a

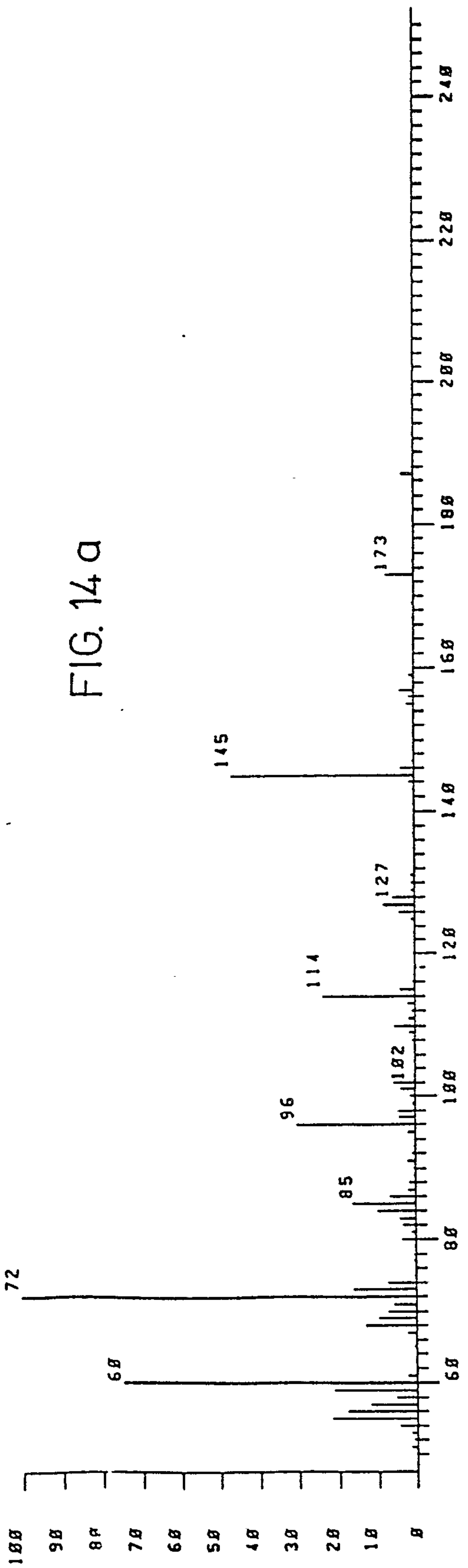


FIG. 14 b

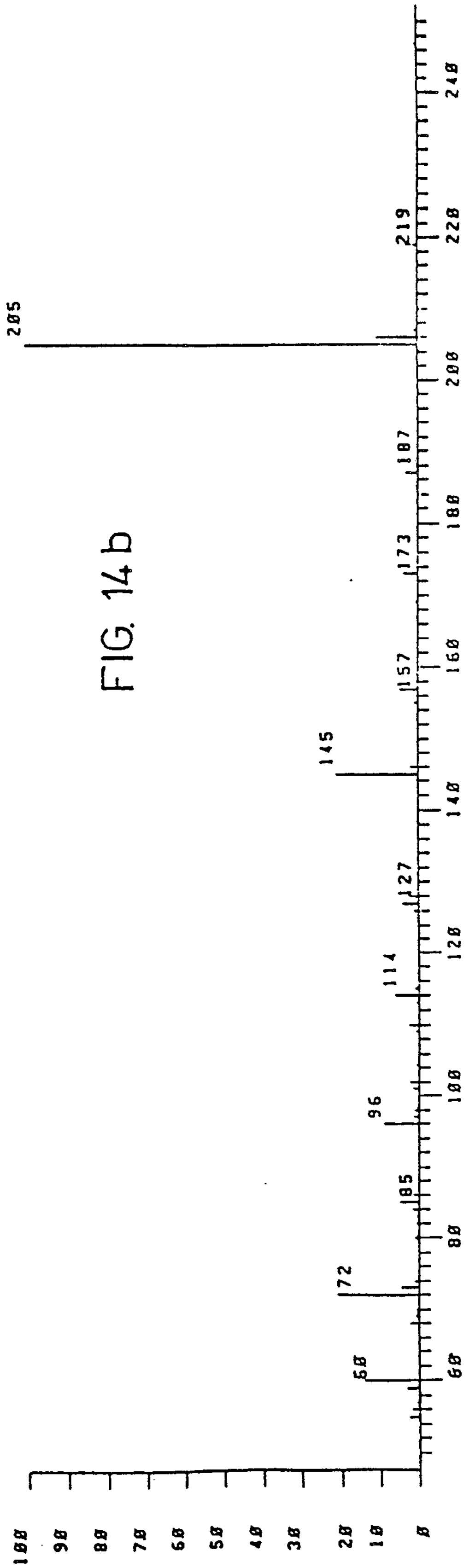

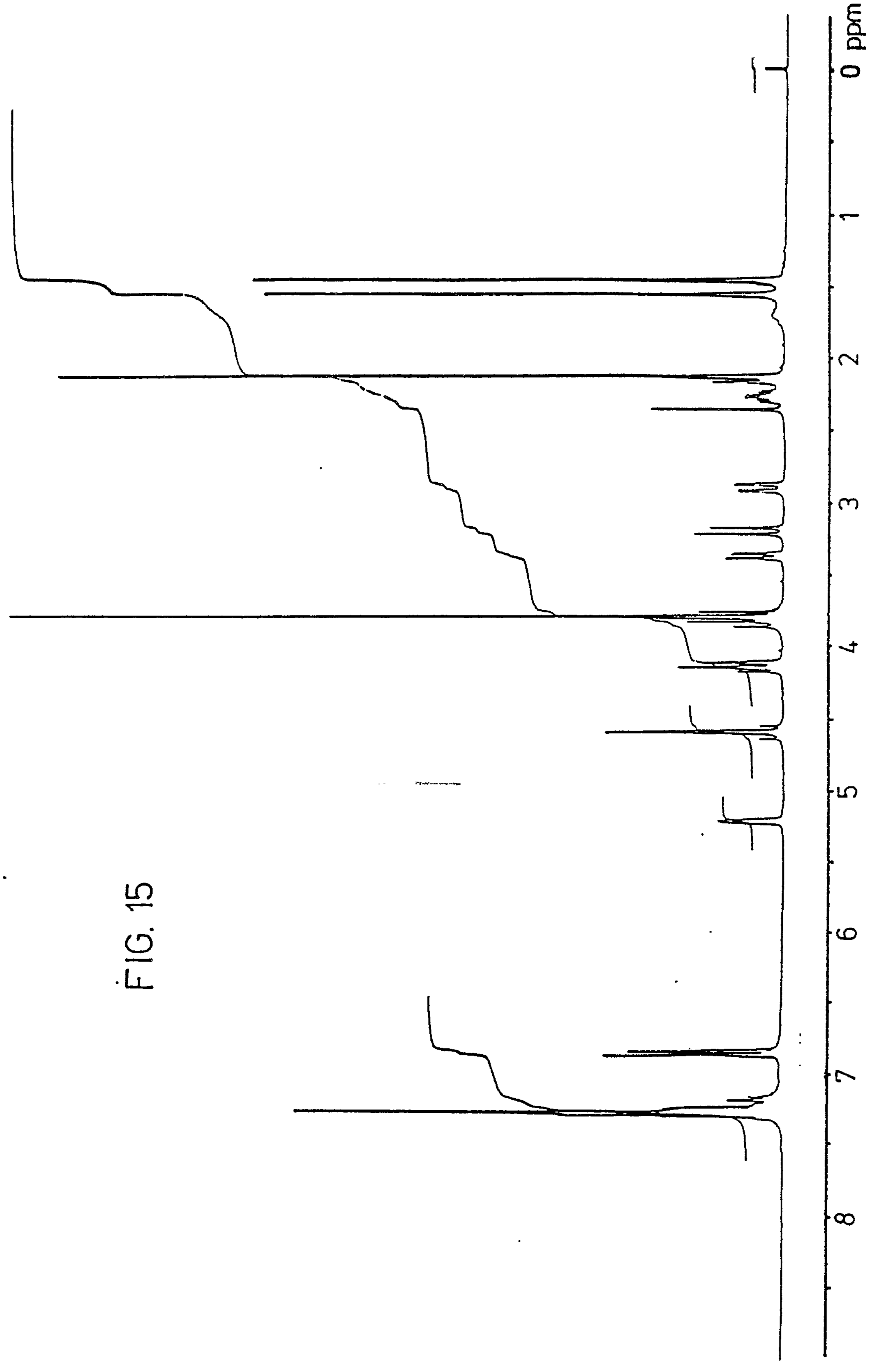


FIG. 15

FIG. 16

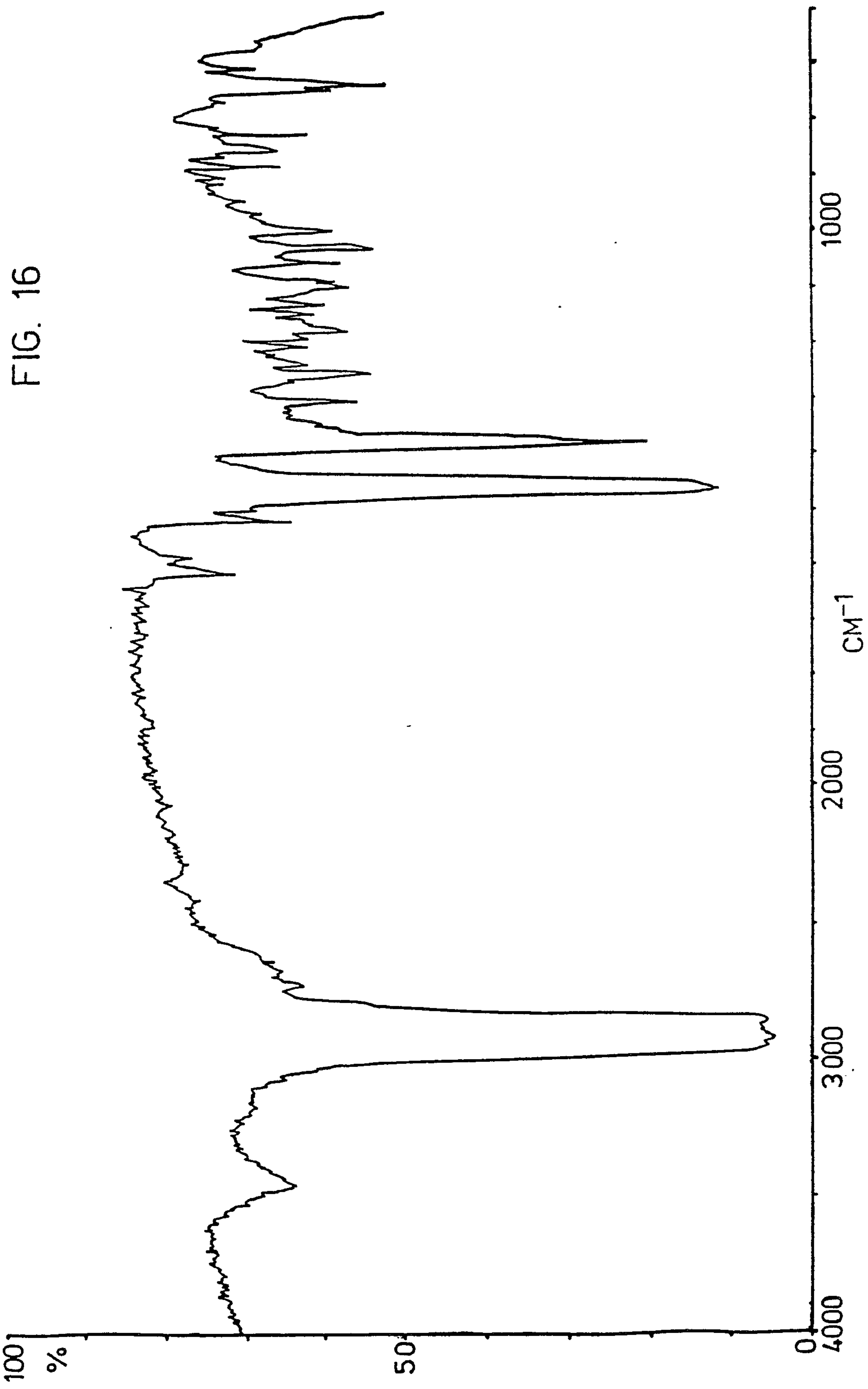

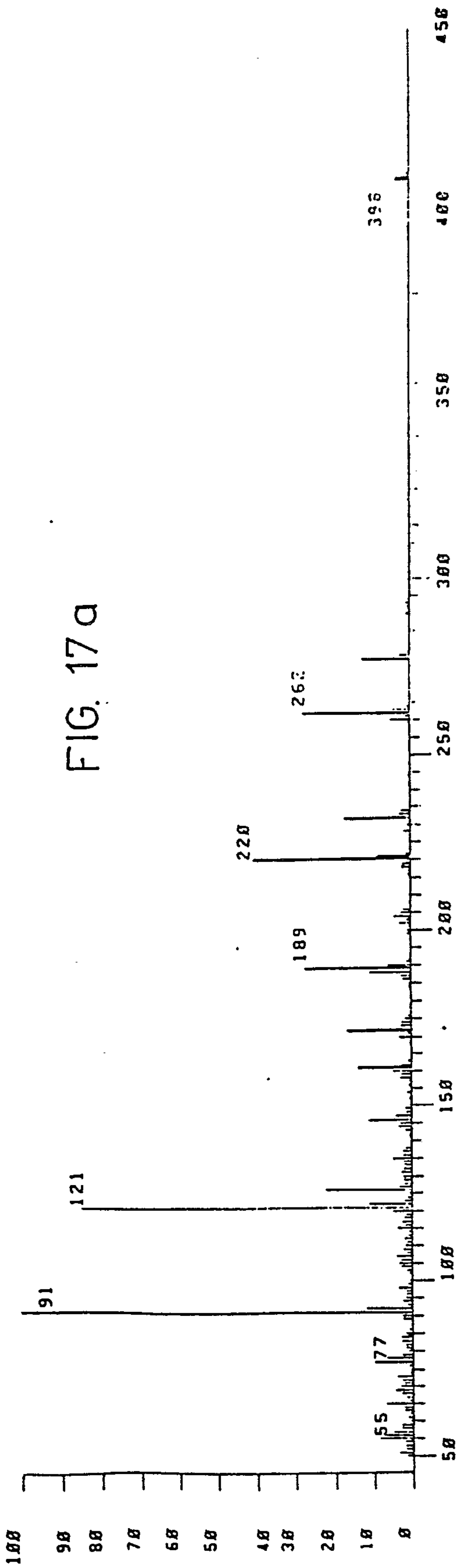


FIG. 17a

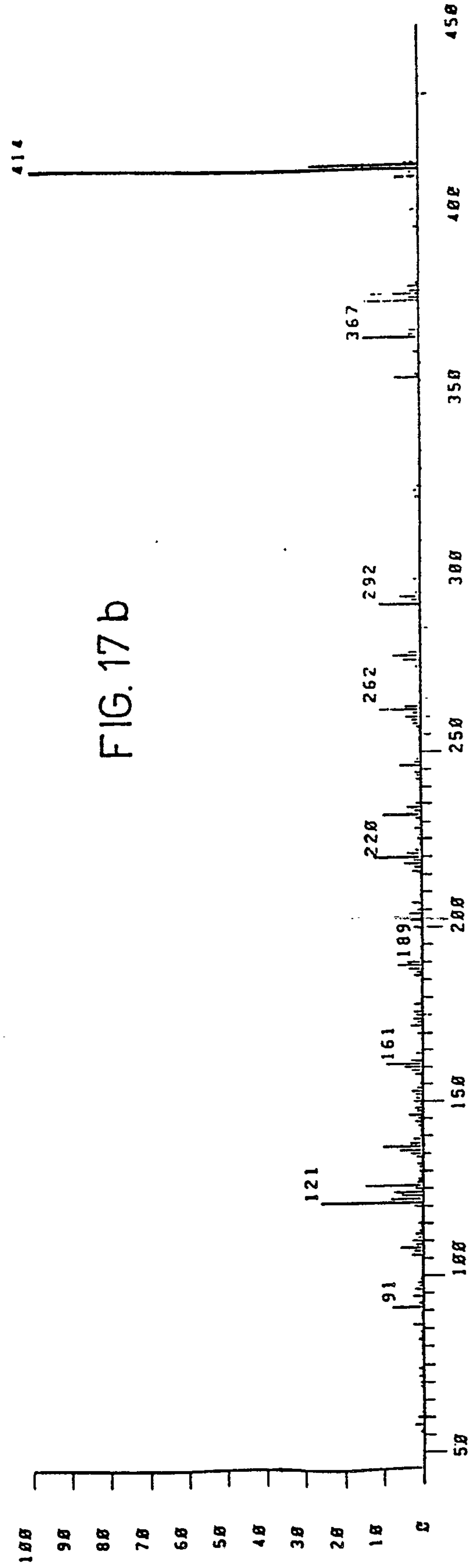


FIG. 17b

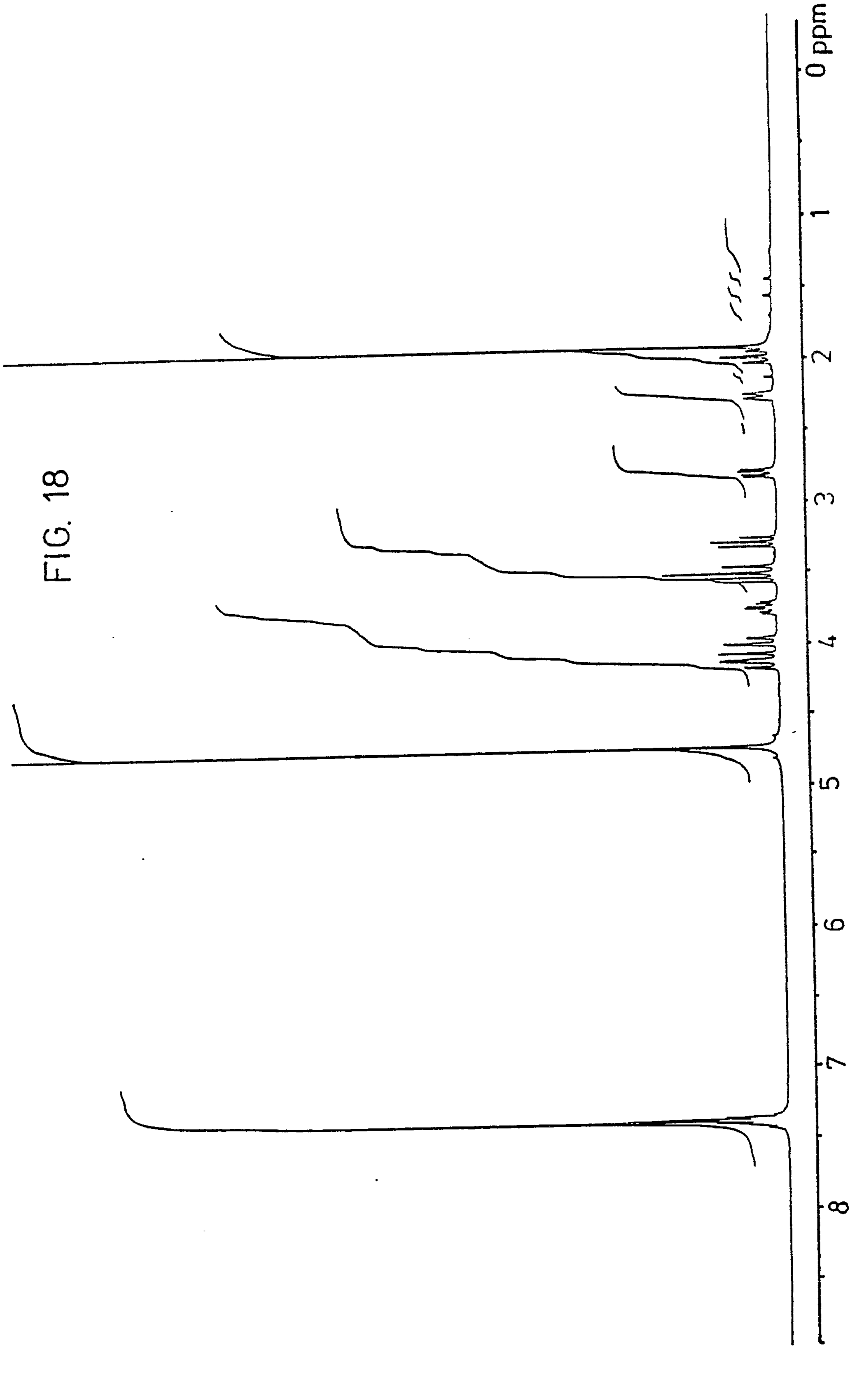


FIG. 18

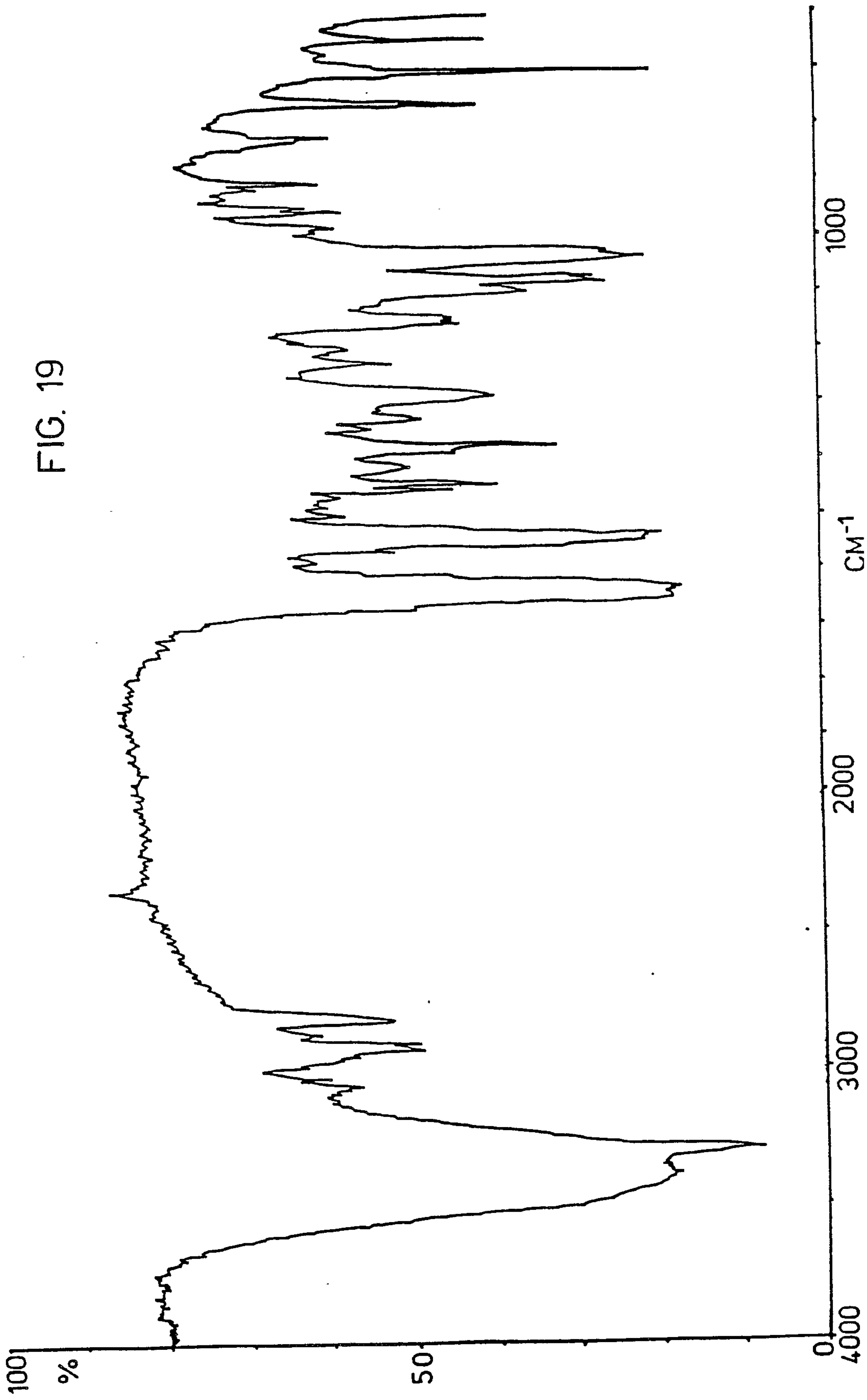


FIG. 19

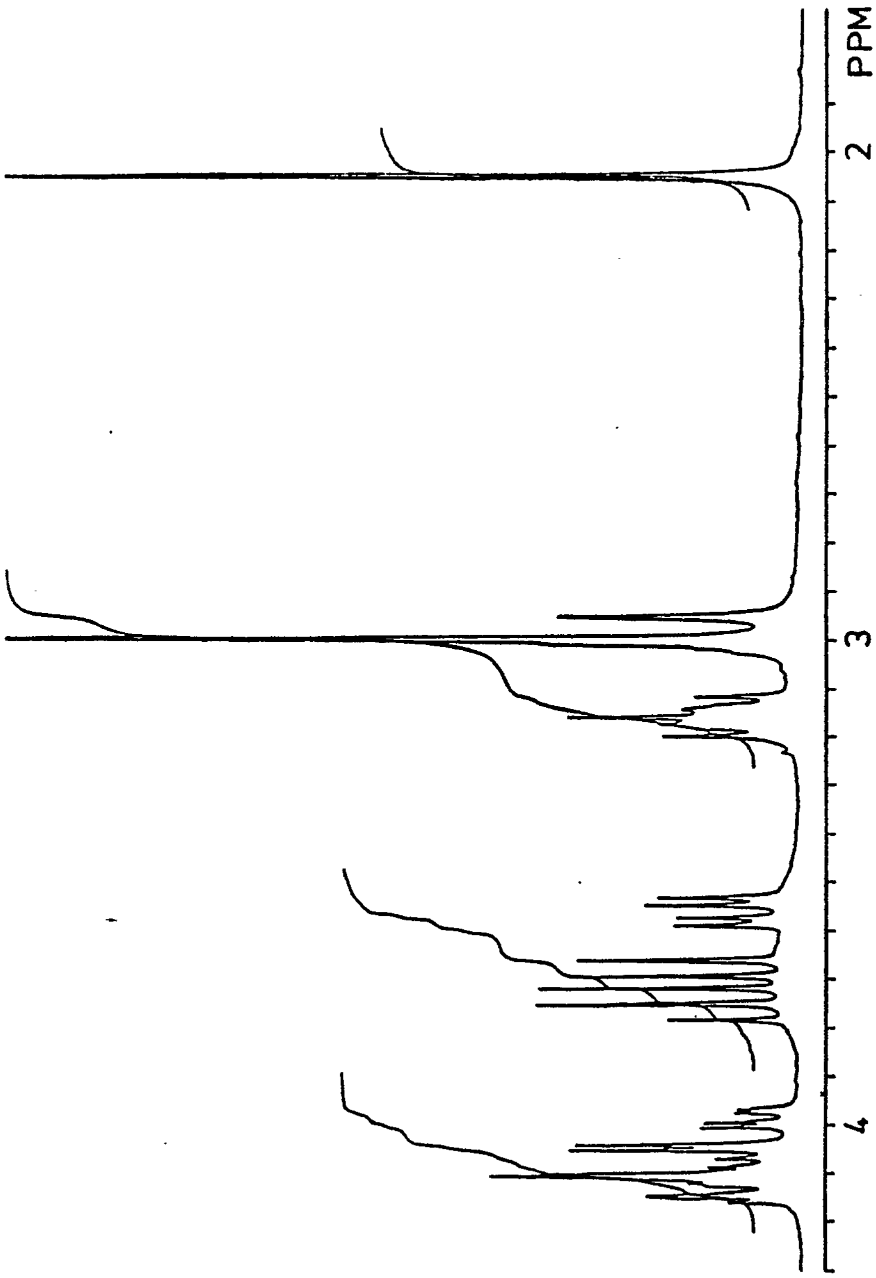


FIG. 20

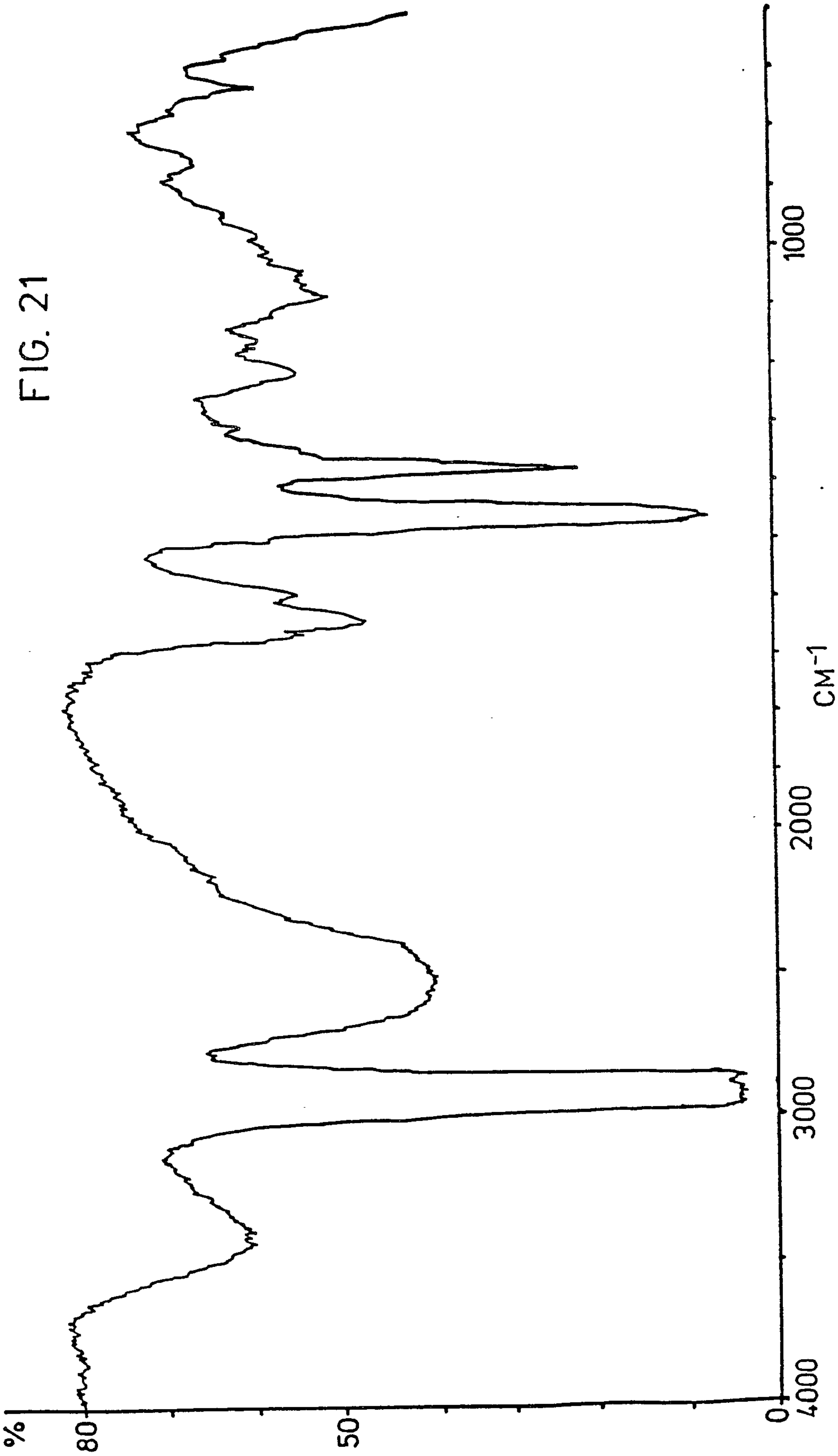


FIG. 21